# EUROPEAN PATENT SPECIFICATION

(11) **EP 2 335 066 B1**
(45) Date of publication and mention of the grant of the patent: **28.12.2016**
(21) Application number: 09815357.0
(22) Date of filing: 21.09.2009
(51) Int. Cl.: G01N 33/48, C12Q 1/68, G01N 33/68

(54) **METHODS FOR PREDICTION OF MULTIPLE SCLEROSIS DISEASE AND THERAPY RESPONSE**
VERFAHREN ZUR PROGNOSE VON MULTIPLER SKLEROSE UND THERAPIEREAKTION
PROCÉDÉ DE PRÉDICTION DE LA SCLÉROSE EN PLAQUES ET DE LA RÉPONSE À LA THÉRAPIE

(30) Priority: 19.09.2008 US 98650 P
(43) Date of publication of application: 22.06.2011
(73) Proprietor: University of Utah Research Foundation, Salt Lake City, UT 84098 (US); Lineagen, Inc., Salt Lake City, UT 84109 (US)
(72) Inventor: ROSE, John W., Salt Lake City, Utah 84108 (US); LEPPERT, Mark F., Salt Lake City, Utah 84108 (US); PAUL, Michael S., Salt Lake City Utah 84109 (US); MATSUNAMI, Nori, Salt Lake City Utah 84109 (US)
(74) Representative: Bradley, Adrian
(86) International application number: PCT/US2009/057740
(87) International publication number: WO 2010/033951

(56) References cited:
- WO-A1-03/102227
- WO-A2-03/014319
- WO-A2-2005/054810
- WO-A2-2006/097463
- US-A1- 2004 181 048
- US-A1- 2006 198 822
- US-A1- 2006 240 463
- Affymetrix: "README: Genome-Wide Human SNP Array 6.0 Sample Data Set", , 25 May 2007 (2007-05-25), pages 1-4, XP55004755, Retrieved from the Internet: URL:http://media.affymetrix.com/support/te chnical/other/genome_wide_snp6_sample_data set_readme.pdf [retrieved on 2011-08-12]
- KORDONOURI ET AL.: 'Natural course of autoimmune thyroiditis in type 1 diabetes: association with gender, age, diabetes duration, and puberty' ARCH DIS CHILD. vol. 90, no. 4, 2005, pages 411 - 414, XP008145557
- CARACCIO ET AL.: 'Long-Term Follow-Up of 106 Multiple Sclerosis Patients Undergoing Interferon- 1a a or 1b Therapy: Predictive Factors of Thyroid Disease Development and Duration.' J CLIN ENDOCRINOL METAB. vol. 90, no. 7, 2005, pages 4133 - 4137, XP008145551
- AMOS ET AL.: 'High-density SNP analysis of 642 Caucasian families with rheumatoid arthritis identifies two new linkage regions on 11p12 and 2q33.' GENES IMMUN. vol. 7, no. 4, 2006, pages 277 - 286, XP008145556
- AL591888, Human DNA sequence from clone RP11-333H8 on chromosome 1, complete sequence, 13 June 2009, [online]. [Retrieved on 2010.02.16]. Retrieved from the Intemet: <URL: http://www.ncbi.nlm.nih.gov/nuccore/1572215 7>, 100% between 87065-87116 XP008145555

### SEQUENCE LISTING

<110> University of Utah
<120> METHODS FOR IDENTIFICATION AND PREDICTION OF MULTIPLE SCLEROSIS DISEASE AND THERAPY RESPONSE
<130> 39865/3
<150> US 61/098650
   <151> 2008-09-19
<160> 171
<170> PatentIn version 3.5
<210> 1
   <211> 52
   <212> DNA
   <213> Homo Sapiens
<400> 1
   tatggtaaag gacaaccact ctaactstaa ttcacatgtt tattctaagg ta
   52
<210> 2
   <211> 52
   <212> DNA
   <213> Homo Sapiens
<400> 2
   tctcttgttt ttattctaca aaacttycac tgaagctaat attttgatta gc
   52
<210> 3
   <211> 52
   <212> DNA
   <213> Homo Sapiens
<400> 3
   gaatcttgaa agcaatgaga aagaagygat tcatcataca caaaagatcc tt
   52
<210> 4
   <211> 52
   <212> DNA
   <213> Homo Sapiens
<400> 4
   agcaggggaa agtaaatcag gtgagargca tgataaggta caccagagat gg
   52
<210> 5
   <211> 52
   <212> DNA
   <213> Homo Sapiens
<400> 5
   tgtgctcaaa attctgcagt aacctcrtat ttcagtcaga gtaaaagcca aa
   52
<210> 6
   <211> 52
   <212> DNA
   <213> Homo Sapiens
<400> 6
   tgagattaca ggtgtgttct accgcamgta accaatctat tttaattttg ta
   52
<210> 7
   <211> 52
   <212> DNA
   <213> Homo Sapiens
<400> 7
   aaaaagcaat tccctcaaag acggttwtgt aagatgtatg gataaattcc aa
   52
<210> 8
   <211> 52
   <212> DNA
   <213> Homo Sapiens
<400> 8
   tgcattctaa tcctcaatac caattarcac attttatgac tctatttgga ta
   52
<210> 9
   <211> 52
   <212> DNA
   <213> Homo Sapiens
<400> 9
   gtcagaagaa aaggatttta tgactcrcag tacaacaggc agctcttact tc
   52
<210> 10
   <211> 52
   <212> DNA
   <213> Homo Sapiens
<400> 10
   ccaggaacca cagagccctg aagaggrcat cacaaccttg gctctaggat cc
   52
<210> 11
   <211> 52
   <212> DNA
   <213> Homo Sapiens
<400> 11
   tgctctgtaa gccagctaga agccgayggt cagtactcgt ttatcaggaa gg
   52
<210> 12
   <211> 52
   <212> DNA
   <213> Homo Sapiens
<400> 12
   tgaaagacac tcaaaagtgg cacaaayggt ataactgcta tacagggata tc
   52
<210> 13
   <211> 52
   <212> DNA
   <213> Homo Sapiens
<400> 13
   ttgtctcacc attcactggt gctctgmgta tgtattctat aattctactt at
   52
<210> 14
   <211> 52
   <212> DNA
   <213> Homo Sapiens
<400> 14
   tgccctatcc acacgcacat ccaatcraac aactatctac acagaaaaag ca
   52
<210> 15
   <211> 52
   <212> DNA
   <213> Homo Sapiens
<400> 15
   aaatatatta aacaaactgc tttaatwtca acttttttaa tctgaaatga ac
   52
<210> 16
   <211> 52
   <212> DNA
   <213> Homo Sapiens
<400> 16
   cagggagtat aatgctggca tctttayagt ttcggtggag gcctcaggaa at
   52
<210> 17
   <211> 52
   <212> DNA
   <213> Homo Sapiens
<400> 17
   cttatttatt gctgtatcct tagtgasaga acagtggctg gcatttttat tt
   52
<210> 18
   <211> 52
   <212> DNA
   <213> Homo Sapiens
<400> 18
   acatgcaata agaaagctaa taaaaaytca acacttctac tttgtccccc ac
   52
<210> 19
   <211> 52
   <212> DNA
   <213> Homo Sapiens
<400> 19
   atgtgatttt taaaaatcag aaatacrgag tgtcttgtag cataaccaac ca
   52
<210> 20
   <211> 52
   <212> DNA
   <213> Homo Sapiens
<400> 20
   tagaagcaga tccttttttg tatgagyctc agatgacaca gccctttttg ac
   52
<210> 21
   <211> 52
   <212> DNA
   <213> Homo Sapiens
<400> 21
   gtgcgtttta ttgacatgga ttcaaargat ttagaaggat actgaacaaa cg
   52
<210> 22
   <211> 52
   <212> DNA
   <213> Homo Sapiens
<400> 22
   tcaagcatga ctgccaacaa attacaktcc agatttaagc tgcctgagat ga
   52
<210> 23
   <211> 52
   <212> DNA
   <213> Homo Sapiens
<400> 23
   ctgctggcct tttcactggt ttaaagmgtt cccctctgga ggacactaca at
   52
<210> 24
   <211> 52
   <212> DNA
   <213> Homo Sapiens
<400> 24
   ttcttctctc cctgtcttga taaattkggg gaagatctca atgaacaagt gt
   52
<210> 25
   <211> 52
   <212> DNA
   <213> Homo Sapiens
<400> 25
   aacaaatttt aattcccttt gttcttyatg agtaaataca aatttttctc tc
   52
<210> 26
   <211> 52
   <212> DNA
   <213> Homo Sapiens
<400> 26
   ggcacagagc tcctaaaacc cttgaamaga tgatccttaa cttatgatgg gg
   52
<210> 27
   <211> 52
   <212> DNA
   <213> Homo Sapiens
<400> 27
   attttgaggc tgatgtttcc atgcatyaat gtgagtctgt ctaaaagagg at
   52
<210> 28
   <211> 52
   <212> DNA
   <213> Homo Sapiens
<400> 28
   tatgaatgta ccataaattg gcagacwttg ggtcatttcc aagctattgt tt
   52
<210> 29
   <211> 52
   <212> DNA
   <213> Homo Sapiens
<400> 29
   tttcttgcat actgccttga ttctttrtgc tgttctattc tgtctatgct tg
   52
<210> 30
   <211> 52
   <212> DNA
   <213> Homo Sapiens
<400> 30
   tgagagctga gttgggagga ggcacayttc ttccatagta ataacaggga ac
   52
<210> 31
   <211> 52
   <212> DNA
   <213> Homo Sapiens
<400> 31
   gcctacatag ttttgcagtt ttggaartcc aggttttgtt ctggttttta tt
   52
<210> 32
   <211> 52
   <212> DNA
   <213> Homo Sapiens
<400> 32
   gtgtactcat ggatattgac agcgtamttt tgaaattaag gagggttttt tt
   52
<210> 33
   <211> 52
   <212> DNA
   <213> Homo Sapiens
<400> 33
   gtgtgttggt gtctaattcc ttggccrtcc tttccagatc aatgaccagg ct
   52
<210> 34
   <211> 52
   <212> DNA
   <213> Homo Sapiens
<400> 34
   ccaattcact cttggaggcc actctgrggt gccaccttga gaactggggg aa
   52
<210> 35
   <211> 52
   <212> DNA
   <213> Homo Sapiens
<400> 35
   aagaagcaat tcactgactc ctttctycaa ctggggataa ataacctcta at
   52
<210> 36
   <211> 52
   <212> DNA
   <213> Homo Sapiens
<400> 36
   ccctatcacc tttgccttat tccattsatt agaagcaagt cacagatccc ac
   52
<210> 37
   <211> 52
   <212> DNA
   <213> Homo Sapiens
<400> 37
   agtgtctggc tcatagctgg tgtccartaa aattttaaat gtatgtataa gt
   52
<210> 38
   <211> 52
   <212> DNA
   <213> Homo Sapiens
<400> 38
   accttataat cagtatttat tgagccyttg ccaaagtagt caataccata ct
   52
<210> 39
   <211> 52
   <212> DNA
   <213> Homo Sapiens
<400> 39
   gattacaggc ataagccatc ggccccrgct cattcaagtt ttaaacatta tt
   52
<210> 40
   <211> 52
   <212> DNA
   <213> Homo Sapiens
<400> 40
   tgaaaagtga aggcagaaaa gtttgcrgaa atcagatgag aattttagtc ag
   52
<210> 41
   <211> 52
   <212> DNA
   <213> Homo Sapiens
<400> 41
   cacatttatt ttcctccaat tcttacrtgt ccaaagacag ggaaccacag ac
   52
<210> 42
   <211> 52
   <212> DNA
   <213> Homo Sapiens
<400> 42
   tccagcatca gccccaacac cagagamaga acctgaagag ccagcacagg cc
   52
<210> 43
   <211> 52
   <212> DNA
   <213> Homo Sapiens
<400> 43
   gcaacgtaga aaggcttgat tataaayaca taccaggcag aaaaaaatta tt
   52
<210> 44
   <211> 52
   <212> DNA
   <213> Homo Sapiens
<400> 44
   ttccagtgac accaaggcac tctacarggc aagtattgct ttgttttcca tc
   52
<210> 45
   <211> 52
   <212> DNA
   <213> Homo Sapiens
<400> 45
   ctgggatcac ctaaattaac tacctayggc caagtcctgt ctcaagctct gc
   52
<210> 46
   <211> 52
   <212> DNA
   <213> Homo Sapiens
<400> 46
   ctctgagcac ttcagagatc tttctayagt cctacatttg acacgtggaa ac
   52
<210> 47
   <211> 52
   <212> DNA
   <213> Homo Sapiens
<400> 47
   ccagcccttt ctgctctgtc ctcacaygta ctgcaggtac actgggctgt aa
   52
<210> 48
   <211> 52
   <212> DNA
   <213> Homo Sapiens
<400> 48
   gcccagtctg gccctaactt aagatcratt tctgattata atcataatca ga
   52
<210> 49
   <211> 52
   <212> DNA
   <213> Homo Sapiens
<400> 49
   agagcacaat ccttgaagcg ttttaaygtg ggacagcctc cctcatctat gc
   52
<210> 50
   <211> 52
   <212> DNA
   <213> Homo Sapiens
<400> 50
   caagtctgtt ctctacctct ctgataycat ttatttaccc tgctcctcac cc
   52
<210> 51
   <211> 52
   <212> DNA
   <213> Homo Sapiens
<400> 51
   acgtctgcat gtaaaataca ggttggwaaa gcatttgatt agctgagttg ag
   52
<210> 52
   <211> 52
   <212> DNA
   <213> Homo Sapiens
<400> 52
   tttggtactt atttttgaaa ctacagktgc catctcttat tgacctctca gt
   52
<210> 53
   <211> 52
   <212> DNA
   <213> Homo Sapiens
<400> 53
   ttgggtaaca ctgtcaacta actaagycta acggacatct ataaaacact ct
   52
<210> 54
   <211> 52
   <212> DNA
   <213> Homo Sapiens
<400> 54
   acatattaga actattataa aatcttyaat cttatgccct tttaatgtga tt
   52
<210> 55
   <211> 52
   <212> DNA
   <213> Homo Sapiens
<400> 55
   taagtgatta cagaataggc tgattgytat agggttgccc accagcctca gg
   52
<210> 56
   <211> 52
   <212> DNA
   <213> Homo Sapiens
<400> 56
   tgtcttgtcc tgcggtgttc tcctctyttg aggacccagg attcagtgta aa
   52
<210> 57
   <211> 52
   <212> DNA
   <213> Homo Sapiens
<400> 57
   aaactaacat ctttaaaaaa atttcartgg gcaagtgtgt ctgttttcct aa
   52
<210> 58
   <211> 52
   <212> DNA
   <213> Homo Sapiens
<400> 58
   cagttgcacc cacaattcaa acatccsctt actagatcaa attaactctg cc
   52
<210> 59
   <211> 52
   <212> DNA
   <213> Homo Sapiens
<400> 59
   gacataagtc attaacttct tctaatstgt gactaagatg tgagtcctga ag
   52
<210> 60
   <211> 52
   <212> DNA
   <213> Homo Sapiens
<400> 60
   ggtggagggt gagaggacgt agacagwcag gaaaaataac taaggggttc ta
   52
<210> 61
   <211> 52
   <212> DNA
   <213> Homo Sapiens
<400> 61
   gaaattaggc ccctctgagt aaagccrgta gcagaaaata tactgcctcc cc
   52
<210> 62
   <211> 52
   <212> DNA
   <213> Homo Sapiens
<400> 62
   tatatttttc atttatagat attgccmggt aacacttttt aaacttttat tt
   52
<210> 63
   <211> 52
   <212> DNA
   <213> Homo Sapiens
<400> 63
   aagtgatttg ataattaaag ctcatgktat atcacatcct gtgtctactc ct
   52
<210> 64
   <211> 52
   <212> DNA
   <213> Homo Sapiens
<400> 64
   atcctgtaga gcaatggttc ttaaackttg gtatgcacgc aaatcacctg gg
   52
<210> 66
   <211> 52
   <212> DNA
   <213> Homo Sapiens
<400> 66
   tttgcctgtg gcagaaatca gtaattkatc atgattctct ttcctgctca gc
   52
<210> 67
   <211> 52
   <212> DNA
   <213> Homo Sapiens
<400> 67
   tgagaaatag aaaataaact gaaatayggt tcctggaatt ggcactgagg aa
   52
<210> 68
   <211> 52
   <212> DNA
   <213> Homo Sapiens
<400> 68
   ccagggaggg atttttgggt agctcayctt tgtgtggttg acagggttgt tt
   52
<210> 69
   <211> 52
   <212> DNA
   <213> Homo Sapiens
<400> 69
   gcactggtta aatctggtat tctggtrgac tgttactgga gaggtttttt cc
   52
<210> 70
   <211> 52
   <212> DNA
   <213> Homo Sapiens
<400> 70
   cttcacatcc tgaagttggt actcttstca atcataatta ccctaactgt gt
   52
<210> 71
   <211> 52
   <212> DNA
   <213> Homo Sapiens
<400> 71
   aaatcttgag agggatatga acatccrgat tgaagacgat caaagcatcc ca
   52
<210> 72
   <211> 52
   <212> DNA
   <213> Homo Sapiens
<400> 72
   aagagagaat tctgaaagaa acatcaygtg taagagatct cccataagtc ca
   52
<210> 73
   <211> 52
   <212> DNA
   <213> Homo Sapiens
<400> 73
   tactgttaag agttatccag gcaatcrttg acagtacctt tgtctagttt gt
   52
<210> 74
   <211> 52
   <212> DNA
   <213> Homo Sapiens
<400> 74
   aggccagttt ggatctgaag gtggtargta agaattctag atagatattt tg
   52
<210> 75
   <211> 52
   <212> DNA
   <213> Homo Sapiens
<400> 75
   accaccttca gatccaaact ggcctcytgg tagacatcat ctttttcaaa aa
   52
<210> 76
   <211> 52
   <212> DNA
   <213> Homo Sapiens
<400> 76
   cttgccaagt ctcccatctt cccaacytct aaatgactga gggcccagga ct
   52
<210> 77
   <211> 52
   <212> DNA
   <213> Homo Sapiens
<400> 77
   agtctaatat gtggaataag gtgaaayaga aacagatcat ccttcatagg aa
   52
<210> 78
   <211> 52
   <212> DNA
   <213> Homo Sapiens
<400> 78
   atttatcatt gttaccagca agagggktat ttcaatacaa actactccgt ca
   52
<210> 79
   <211> 52
   <212> DNA
   <213> Homo Sapiens
<400> 79
   aattctggac caccttcatt ttccaaytta tttcattgtg ttgtgataag aa
   52
<210> 80
   <211> 52
   <212> DNA
   <213> Homo Sapiens
<400> 80
   gcccccagtc tctggcaacc accaacstat tctctgtttc tatgagattg gc
   52
<210> 81
   <211> 52
   <212> DNA
   <213> Homo Sapiens
<400> 81
   gaataggaag aattgtacct acttcawctt gtcttagaac cagaaatctc tc
   52
<210> 82
   <211> 52
   <212> DNA
   <213> Homo Sapiens
<400> 82
   ccttttctct ggacatatgc agcattrttt ggaaatcttt tttcattaag ca
   52
<210> 83
   <211> 52
   <212> DNA
   <213> Homo Sapiens
<400> 83
   accagcttct gtgacctctc aggatgmgaa aaagtaacac aagaaaagct tc
   52
<210> 84
   <211> 52
   <212> DNA
   <213> Homo Sapiens
<400> 84
   gcttgctctg ccccaggttt ctgaacrtgg aatacatttc tccagggaaa ct
   52
<210> 85
   <211> 52
   <212> DNA
   <213> Homo Sapiens
<400> 85
   aaggaaatgg ggaatctgat attattstct ctcatctcca gagcaacatt gg
   52
<210> 86
   <211> 52
   <212> DNA
   <213> Homo Sapiens
<400> 86
   tcactgaaga aacttctgct ttaatgrctt tacaaagctg gcaatattac aa
   52
<210> 87
   <211> 52
   <212> DNA
   <213> Homo Sapiens
<400> 87
   cagctccgcc accgcccgga actttcygac gtccctatgg aagcgcgcat ac
   52
<210> 88
   <211> 52
   <212> DNA
   <213> Homo Sapiens
<400> 88
   aatgtagaat tactgcagaa atctgargca ttttactgaa aaatatttgg ga
   52
<210> 89
   <211> 52
   <212> DNA
   <213> Homo Sapiens
<400> 89
   ggatagtcgt caccatttat gacttayaat taccagttgt tgaaagttaa ta
   52
<210> 90
   <211> 52
   <212> DNA
   <213> Homo Sapiens
<400> 90
   aaatgaagat cttgaacctg cattgaytga atggattcat caagaaagca gt
   52
<210> 91
   <211> 52
   <212> DNA
   <213> Homo Sapiens
<400> 91
   gaagttgccg tcactgtagc ttgcatrgtt agcactgcag tctatgctca tg
   52
<210> 92
   <211> 52
   <212> DNA
   <213> Homo Sapiens
<400> 92
   aatgagcagg tcccagactt tgagtaygac ctttgcaaag ctctctttgt cc
   52
<210> 93
   <211> 52
   <212> DNA
   <213> Homo Sapiens
<400> 93
   aaatgttcat cagtggtcac aaatatmatg tatctaaaat agggacagta ag
   52
<210> 94
   <211> 52
   <212> DNA
   <213> Homo Sapiens
<400> 94
   agtaaacagt gcctagatca taagatkcaa ctggcacatc ctgaaactca ct
   52
<210> 95
   <211> 52
   <212> DNA
   <213> Homo Sapiens
<400> 95
   cagagatccc taggtaggtg gggtcarctt aacatttgga gaattccata cg
   52
<210> 96
   <211> 52
   <212> DNA
   <213> Homo Sapiens
<400> 96
   ccatatgcac agatctttgt tagtcayatc tgctcatgga ctcaacaaac ag
   52
<210> 97
   <211> 52
   <212> DNA
   <213> Homo Sapiens
<400> 97
   gggaacacaa gtgcctgagt tagattsatc aaaaagataa aatttgttat ca
   52
<210> 98
   <211> 52
   <212> DNA
   <213> Homo Sapiens
<400> 98
   catcatattc tgaacacatg gaaggamgag tacgggaaat aagagtggct gt
   52
<210> 99
   <211> 52
   <212> DNA
   <213> Homo Sapiens
<400> 99
   gtatgctttc accaacttcc ttcaccycaa cttatggtgc agaatttcta cc
   52
<210> 100
   <211> 52
   <212> DNA
   <213> Homo Sapiens
<400> 100
   aactaacttg tgctgtccct ggttccyacc tttttccatg gctcaatatc aa
   52
<210> 101
   <211> 52
   <212> DNA
   <213> Homo Sapiens
<400> 101
   aattatggtg attctgctcc atagcarctt cattaaagga cctagtctaa gt
   52
<210> 102
   <211> 52
   <212> DNA
   <213> Homo Sapiens
<400> 102
   ctcttctgta ataaataaat aaattgwgac ttgaactcct actctagttg gc
   52
<210> 103
   <211> 52
   <212> DNA
   <213> Homo Sapiens
<400> 103
   aaattgatct tcttcttggg aattttygtt cagcacactg agtcccaatg gg
   52
<210> 104
   <211> 52
   <212> DNA
   <213> Homo Sapiens
<400> 104
   cgagattgtt gatttgaagg tcttgtrtga taaagcattg tatttctgta ga
   52
<210> 105
   <211> 52
   <212> DNA
   <213> Homo Sapiens
<400> 105
   atctgctttc accatagccc caggttrctc ttcagctaaa cagaataggt ta
   52
<210> 106
   <211> 52
   <212> DNA
   <213> Homo Sapiens
<400> 106
   cttagttaac aactttgcag ctaagtsctc tcaacatgtt tttgaccgcg tt
   52
<210> 107
   <211> 52
   <212> DNA
   <213> Homo Sapiens
<400> 107
   aggctgtcga tatcctcctc acagaaytcc ccaaagtaca ttgtatttgc tc
   52
<210> 108
   <211> 52
   <212> DNA
   <213> Homo Sapiens
<400> 108
   gcaatgacag aaattaaaaa accagtygag cttccacttt tcatttcaga ag
   52
<210> 109
   <211> 52
   <212> DNA
   <213> Homo Sapiens
<400> 109
   gttgctattc ttgtaatagt ctttctygta ccctataatt gttagaagaa at
   52
<210> 110
   <211> 52
   <212> DNA
   <213> Homo Sapiens
<400> 110
   actcttatgt tttaggccaa tttcatrtca ttccccagat catatttcaa gt
   52
<210> 111
   <211> 52
   <212> DNA
   <213> Homo Sapiens
<400> 111
   catatcattc cccagatcat atttcawgtc cagtaacaca ggagcaacca ag
   52
<210> 112
   <211> 52
   <212> DNA
   <213> Homo Sapiens
<400> 112
   tgtaagaacc tctcaggaat gcaactrtaa agaatgtgta tgcaggaact aa
   52
<210> 113
   <211> 52
   <212> DNA
   <213> Homo Sapiens
<400> 113
   cggccagcag ctgcgaaacc cgtccamgcg aaattgagtt cttggctggg cc
   52
<210> 114
   <211> 52
   <212> DNA
   <213> Homo Sapiens
<400> 114
   agtgaagagg gcagtcggac cgattcraca ttgacctctg ctcttagatc ag
   52
<210> 115
   <211> 52
   <212> DNA
   <213> Homo Sapiens
<400> 115
   ggcgaagact tgtgtgatgc atgcagrgac tcaaatttag tgtcctctga ac
   52
<210> 116
   <211> 52
   <212> DNA
   <213> Homo Sapiens
<400> 116
   agtgagcctg caaatggtag tttgggygaa gacttgtgtg atgcatgcag gg
   52
<210> 117
   <211> 52
   <212> DNA
   <213> Homo Sapiens
<400> 117
   cagaattgaa ttgatttaga ggaaccmggt gtctgctgga gaattgcttg gt
   52
<210> 118
   <211> 52
   <212> DNA
   <213> Homo Sapiens
<400> 118
   tgacttcctc tctccccaaa gttcttstta acttctgagt atgacttagc tt
   52
<210> 119
   <211> 52
   <212> DNA
   <213> Homo Sapiens
<400> 119
   tggccttttc tctctctatc tgaagaygtg gcttactgta acaggaacta tc
   52
<210> 120
   <211> 52
   <212> DNA
   <213> Homo Sapiens
<400> 120
   attagcccta gggaaactgt tggtccygaa atctgtaggg tggtcttgcc ca
   52
<210> 121
   <211> 52
   <212> DNA
   <213> Homo Sapiens
<400> 121
   atcatatgaa ttttgggacg gcatatwcag tccacaccag gaagttcttg ca
   52
<210> 122
   <211> 52
   <212> DNA
   <213> Homo Sapiens
<400> 122
   gagatcagtg tgtcagcctg gtagggwtct ggtgaaggcc tgctggcagc at
   52
<210> 123
   <211> 52
   <212> DNA
   <213> Homo Sapiens
<400> 123
   tgcctccacc cagggatagc cacttgkaat ccacatggca attgtgaaac aa
   52
<210> 124
   <211> 52
   <212> DNA
   <213> Homo Sapiens
<400> 124
   cagtcgccca cagtgttagg tgcagtyata tgttgtacag atttgtagcc ta
   52
<210> 125
   <211> 52
   <212> DNA
   <213> Homo Sapiens
<400> 125
   gctaagaaag gaagatgaga ggcctcrctt acctgcattc cttggccagc ag
   52
<210> 126
   <211> 52
   <212> DNA
   <213> Homo Sapiens
<400> 126
   gagtgtgtta cctcctttcc aagctcytcg tgataatgca gacttcctgg ag
   52
<210> 127
   <211> 52
   <212> DNA
   <213> Homo Sapiens
<400> 127
   attcattcat ttctttttaa gtcctgyttc ttctaatcat ttagtctaaa tg
   52
<210> 128
   <211> 52
   <212> DNA
   <213> Homo Sapiens
<400> 128
   gggaaaaaaa tgtgctttgt taggaargat aaattgacac atttaagagc tt
   52
<210> 129
   <211> 52
   <212> DNA
   <213> Homo Sapiens
<400> 129
   gtaagtactg tacttaacac aacttcrttt cacatataaa attattttaa at
   52
<210> 130
   <211> 52
   <212> DNA
   <213> Homo Sapiens
<400> 130
   ctcatgttgt gatattctag attcagrtat ttccctgagc agagaggctg aa
   52
<210> 131
   <211> 52
   <212> DNA
   <213> Homo Sapiens
<400> 131
   aaatataact ctagttaatg gggataygca atttgactgt agaccaataa tt
   52
<210> 132
   <211> 52
   <212> DNA
   <213> Homo Sapiens
<400> 132
   tttgaacccg atgtcctttc tagaggytat ttggaagtga taatatggca tt
   52
<210> 133
   <211> 52
   <212> DNA
   <213> Homo Sapiens
<400> 133
   ggctatttgg aagtgataat atggcaytag aaactataag tgacaaatca aa
   52
<210> 134
   <211> 52
   <212> DNA
   <213> Homo Sapiens
<400> 134
   tagaaatggt tcaatttcag tagactrtgt caaacaccaa ggtaggagta ca
   52
<210> 135
   <211> 52
   <212> DNA
   <213> Homo Sapiens
<400> 135
   taaattttta attattacca tattccrtcc ttagtcaaat ccttaataac tt
   52
<210> 136
   <211> 52
   <212> DNA
   <213> Homo Sapiens
<400> 136
   gaggtagata gaactcatga tacaacrttt ccatttttat tatatgatag ca 52
<210> 137
   <211> 52
   <212> DNA
   <213> Homo Sapiens
<400> 137
   tgaggcatgg caaccttaac taatttrcca agtcccatag ctagaaacag tt 52
<210> 138
   <211> 52
   <212> DNA
   <213> Homo Sapiens
<400> 138
   tattttccat ctccatattt ctgtggygaa ttttgagtga tttcctcaaa ta 52
<210> 139
   <211> 52
   <212> DNA
   <213> Homo Sapiens
<400> 139
   ctaaattata ggacaacatt ttacttyaga acttaatttc atcttaatta tg 52
<210> 140
   <211> 52
   <212> DNA
   <213> Homo Sapiens
<400> 140
   aaaatttaat gtaattaagg tgttctraaa tacctgagct gcttctgatt at 52
<210> 141
   <211> 52
   <212> DNA
   <213> Homo Sapiens
<400> 141
   tattttttta aaagacaagc atattcktaa gtcttccaat gtttaaatct tg 52
<210> 142
   <211> 52
   <212> DNA
   <213> Homo Sapiens
<400> 142
   aaaggagaaa ttcaaggctg caggtcrtaa tctgggagaa caggtgctag gt
   52
<210> 143
   <211> 52
   <212> DNA
   <213> Homo Sapiens
<400> 143
   aatgtagata aatttatgaa ttagacrtcc taaaatcagt aaaaccttag tc
   52
<210> 144
   <211> 52
   <212> DNA
   <213> Homo Sapiens
<400> 144
   catacaaaaa caggtagcag tccaaaytgg gtgcacagcc cataggttgc ca
   52
<210> 145
   <211> 52
   <212> DNA
   <213> Homo Sapiens
<400> 145
   cagtgcaggt ctcttgtagt tcatccyacg tgtagttcat cgaacttttg aa
   52
<210> 146
   <211> 52
   <212> DNA
   <213> Homo Sapiens
<400> 146
   aatataaaac agtatgcctg taggagrata gcagaactgg gtaaattaga aa
   52
<210> 147
   <211> 52
   <212> DNA
   <213> Homo Sapiens
<400> 147
   tttgtcattt gtatagcttc tttggaraaa aatttgttca aatcccttgc ct
   52
<210> 148
   <211> 52
   <212> DNA
   <213> Homo Sapiens
<400> 148
   aaaatcctaa accacaaatc ttctttmaac attctatttc aacactgaga at
   52
<210> 149
   <211> 52
   <212> DNA
   <213> Homo Sapiens
<400> 149
   tatctgaatt gcatgctatc tgtactrttt ggagaaagtt gcaaatcgca ca
   52
<210> 150
   <211> 52
   <212> DNA
   <213> Homo Sapiens
<400> 150
   tggcatattt gttaacctga cctatamaaa ctataacttc ggccaggtgc ag
   52
<210> 151
   <211> 52
   <212> DNA
   <213> Homo Sapiens
<400> 151
   gccagggcaa aatgttaaag aatggcyccg tcacaggcac ccctgtgcag ca
   52
<210> 152
   <211> 52
   <212> DNA
   <213> Homo Sapiens
<400> 152
   ccaaggagag ccttcccaaa tggatgygaa gatcaggact tgcttgcttt cc
   52
<210> 153
   <211> 52
   <212> DNA
   <213> Homo Sapiens
<400> 153
   aatggaaacc tgggtgtaat gagacckgaa aacagaatgg acagataggg gg
   52
<210> 154
   <211> 52
   <212> DNA
   <213> Homo Sapiens
<400> 154
   tttaaagttt ttgttaaaaa tgaagayatg gacacgcaat agcctaggcc ta
   52
<210> 155
   <211> 52
   <212> DNA
   <213> Homo Sapiens
<400> 155
   ggagctccct ggaggaggta gcggacrttt gactggggtt tggcatattc aa
   52
<210> 156
   <211> 52
   <212> DNA
   <213> Homo Sapiens
<400> 156
   ttctctctct acacctcatg gttctcyaaa tggtgaccat ctcagcttcc ag
   52
<210> 157
   <211> 52
   <212> DNA
   <213> Homo Sapiens
<400> 157
   ttgctttcat tgagtgcttc tccactstct gcgattattc tttatttttt at
   52
<210> 158
   <211> 52
   <212> DNA
   <213> Homo Sapiens
<400> 158
   aggaagatgt taccattttc cctttcrgca ggtcagaaag tggcctaccc aa
   52
<210> 159
   <211> 52
   <212> DNA
   <213> Homo Sapiens
<400> 159
   atgtctcact ttgttaactc ctaaaayctt cagtcataac agctaatgtg ag
   52
<210> 160
   <211> 52
   <212> DNA
   <213> Homo Sapiens
<400> 160
   agacccctct ggttgcatac aaacggytca caagatagat ggcctggctt cg
   52
<210> 161
   <211> 52
   <212> DNA
   <213> Homo Sapiens
<400> 161
   tggttggcag cagggaattt catcatstag agggattaga attcacttag cc
   52
<210> 162
   <211> 52
   <212> DNA
   <213> Homo Sapiens
<400> 162
   tgtggcacaa cctaggtttt aacaggrcca ctcagacctc tgcttggaga ac
   52
<210> 163
   <211> 52
   <212> DNA
   <213> Homo Sapiens
<400> 163
   cctacagtga cccttttctc cttgacrtgc cacaagattg gtgaataaac ca
   52
<210> 164
   <211> 52
   <212> DNA
   <213> Homo Sapiens
<400> 164
   ggcatggaag aggatcaacc ccttgtyggg cagcagagga gactgaggca aa
   52
<210> 165
   <211> 52
   <212> DNA
   <213> Homo Sapiens
<400> 165
   aagaactggg ttttcctgtg tttggtyctg cattctaaaa caccactaat ac
   52
<210> 166
   <211> 52
   <212> DNA
   <213> Homo Sapiens
<400> 166
   cttttttcag tgtctttcac agctttygta acttctagct ctaggaaagg gt
   52
<210> 167
   <211> 52
   <212> DNA
   <213> Homo Sapiens
<400> 167
   atagtgggaa gagttactca tataccrtaa cagacactca cagccatctg gc
   52
<210> 168
   <211> 52
   <212> DNA
   <213> Homo Sapiens
<400> 168
   ctataggact gtatgtttgc caaaagrtta ataatcactt gttccttaat at
   52
<210> 169
   <211> 52
   <212> DNA
   <213> Homo Sapiens
<400> 169
   aaaaggttaa taatcacttg ttccttrata ttcataacat ccctctaaag ca
   52
<210> 170
   <211> 52
   <212> DNA
   <213> Homo Sapiens
<400> 170
   tgagtccctc tcattatttc ttctccrggt tttagctcat gtgcaactat tt
   52
<210> 171
   <211> 52
   <212> DNA
   <213> Homo Sapiens
<400> 171
   gagggatatg ataaaaagtt gaacacrata tgtccatttc aacagttgca tc
   52

## Description

### Technical Field

The present invention relates to the use of clinical, genetic, biological and/or immunological markers as prognostic indicators, diagnostic markers, or predictors of multiple sclerosis (MS) disease and MS therapy response.

### Background

MS is an autoimmune disease that affects the central nervous system (CNS). The CNS consists of the brain, spinal cord, and the optic nerves. Surrounding and protecting the nerve fibers of the CNS is a fatty tissue called myelin which helps nerve fibers conduct electrical impulses. In MS, myelin is lost in multiple areas, leaving scar tissue called sclerosis. These damaged areas are also known as plaques or lesions. Sometimes the nerve fiber itself is damaged or broken. When myelin or the nerve fiber is destroyed or damaged, the ability of the nerves to conduct electrical impulses to and from the brain is disrupted, and this produces the various symptoms of MS.

MS is a complex disease with heterogeneous disease course, neuropathology and gender bias. The disorder features autoimmunity, inflammation, neurodegeneration and impaired regeneration. Distinct neuropathologies are now being associated with the progressive and relapsing states of the disease. In terms of etiology, genetics likely plays a role even though MS is not an inherited disease. However, there are several environmental factors such as exposure to certain pathogens or damage mechanism which might increase MS susceptibility.

People with MS can expect one of four clinical courses of disease, each of which might be mild, moderate, or severe. These include Relapsing-Remitting (RR), Primary-Progressive (PP), Secondary-Progressive (SP), and Progressive-Relapsing (PR). Individuals with RR MS experience clearly defined flare-ups (also called relapses, attacks, or exacerbations). These are episodes of acute worsening of neurologic function. They are followed by partial or complete recovery periods (remissions) free of disease progression. Individuals with PP MS experience a slow but nearly continuous worsening of their disease from the onset, with no distinct relapses or remissions. However, there are variations in rates of progression over time, occasional plateaus, and temporary minor improvements. Individuals with SP MS experience an initial period of relapsing-remitting disease, followed by a steadily worsening disease course with or without occasional flare-ups, minor recoveries (remissions), or plateaus. Individuals with PR MS experience a steadily worsening disease from the onset but also have clear acute relapses (attacks or exacerbations), with or without recovery. In contrast to RR MS, the periods between relapses are characterized by continuing disease progression.

Patients can progress rapidly over several months to death, or may have a few relapses and then remain clinically stable for many decades. It is difficult to predict which patients will progress and which will remain relatively stable. Although there are clearly patients in whom the disease remains benign, it is very difficult to predict which course a patient's disease will follow.

At this time, there is no cure for MS. Despite treatment with available agents, a majority of patients eventually progress to a SP stage of disease leading to severe disability. Treatment with interferons and glitiramer acetate (Copaxone®) exhibit moderate efficacy and have to be injected via frequent subcutaneous or intramuscular injections and experience a poor tolerability profile that includes flu-like symptoms. Treatment of MS with natalizumab (Tysabri®), a humanized monoclonal antibody) is more effective and better tolerated but has been currently relegated to second-line in interferon failures based on concerns over life-threatening side effects that occurred in patients receiving combination therapy with interferons.

Currently there are no reliable clinical, genetic, biological and/or immunological markers that accurately diagnose MS, clinically characterize MS, and forecast a response to MS therapy. WO03/014319, for example, discusses polymorphisms thought to play a role in the incidence, detection or treatment of MS, but investigations were restricted to a limited subset of 15 genes and their clinical utility is unclear. Further, WO 03/102227 discusses expression levels of genes between cells and tissues of MS sufferers and non-sufferers but does not disclose any specific genetic markers. While the interferons and Copaxone® are clearly beneficial, the effect of treatment diminishes over time, and a meaningful number of patients do not respond to one or more of the currently available options. In the absence of prognostic clinical, genetic, biological and/or immunological markers of response, clinicians often approach treatment on a trial and error basis, heavily weighing patient tolerance for side effects and choice of administration versus efficacy. The reliable diagnosis and characterization of MS, and a prognostic indicator of the clinical response to one or more therapies for the treatment of MS, would be very valuable.

### Brief Description of the Drawings

FIG. 1 shows the SNP disclosed herein associated with MS and SEQ. ID. NOS.: 1-171 with SNP alleles indicated by brackets within each sequence.
FIG. 2 shows the difference between the positive and negative anti-TG patients.
FIG. 3 shows the difference between the positive and negative anti-TPO patients.
FIG. 4 shows chromosomal regions with significant linkage in MS families.

### Detailed Description

Disclosed are molecules, materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutation of these molecules and compounds may not be explicitly disclosed, each is specifically contemplated and described herein. For example, if a nucleotide or nucleic acid is disclosed and discussed and a number of modifications that can be made to a number of molecules including the nucleotide or nucleic acid are discussed, each and every combination and permutation of nucleotide or nucleic acid and the modifications that are possible are specifically contemplated unless specifically indicated to the contrary. This concept applies to all aspects of this application including, but not limited to, steps in methods of making and using the disclosed molecules and compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

Those skilled in the art will recognize, or be able to ascertain using no more than routine experimentation, many equivalents to the specific embodiments of the method and compositions described herein. Such equivalents are intended to be encompassed by the following claims.

It is understood that the disclosed methods and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

Unless defined otherwise, all technical and scientific terms used herein have the meanings that would be commonly understood by one of skill in the art in the context of the present specification.

It must be noted that as used herein and in the appended claims, the singular forms "a," "an," and "the" include plural reference unless the context clearly dictates otherwise. Thus, for example, reference to "a nucleotide" includes a plurality of such nucleotides, reference to "the nucleotide" is a reference to one or more nucleotides and equivalents thereof known to those skilled in the art, and so forth.

"Optional" or "optionally" means that the subsequently described event, circumstance, or material may or may not occur or be present, and that the description includes instances where the event, circumstance, or material occurs or is present and instances where it does not occur or is not present.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed. It is also understood that the throughout the application, data is provided in a number of different formats, and that this data represents endpoints and starting points and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

As used herein, the term "subject" means any target of administration. The subject can be a vertebrate, for example, a mammal. Thus, the subject can be a human. The term does not denote a particular age or sex. Thus, adult and newborn subjects, as well as fetuses, whether male or female, are intended to be covered. A patient refers to a subject afflicted with a disease or disorder. Unless otherwise specified, the term "patient" includes human and veterinary subjects.

As used herein, the term "biomarker" or "biological marker" means an indicator of a biologic state and may include a characteristic that is objectively measured as an indicator of normal biological processes, pathologic processes, or pharmacologic responses to a therapeutic or other intervention. A biomarker may indicate a change in expression or state of a protein that correlates with the risk or progression of a disease, or with the susceptibility of the disease in an individual. In certain embodiments, a biomarker may include one or more of the following: genes, proteins, glycoproteins, metabolites, cytokines, and antibodies.

has used herein, the term *"in vitro* diagnostic" means diagnostic tests that may be used to detect or indicate the presence of, the predisposition to, or the risk of, diseases, conditions, infections and/or therapeutic responses. An *in vitro* diagnostic may be used in a laboratory or other health professional setting. Alternatively, an *in vitro* diagnostic may be used by a consumer at home. *In vitro* diagnostic products are those reagents, instruments, and systems intended for use in the *in vitro* diagnosis of disease or other conditions, including a determination of the state of health, in order to cure, mitigate, treat, or prevent disease or its sequelae. *In vitro* diagnostic products may be intended for use in the collection, preparation, and examination of specimens taken from the human body and may comprise one or more laboratory tests such as one or more *in vitro* diagnostic tests. As used herein, the term "laboratory test" means one or more medical or laboratory procedures that involve testing samples of blood, urine, or other tissues or substaces in the body.

The methods and *in vitro* diagnostic products described herein may be used for the diagnosis of MS in at-risk patients, patients with non-specific symptoms possibly associated with MS, and/or patients presenting with Clinically Isolated Syndrome. The methods and *in vitro* diagnostic products described herein may also be used for screening for risk of progressing from at-risk, non-specific symptoms possibly associated with MS, and/or Clinically Isolated Syndrome to fully-diagnosed MS and can be used to rule out screening of diseases and disorders that share symptoms with MS. Further, the methods and *in vitro* diagnostic products described herein may indicate diagnostic information to be included in the current diagnostic evaluation in patients suspected of having MS.

A drug or pharmaceutical agent means any substance used in the prevention, diagnosis, alleviation, treatment or cure of a disease. These terms include a vaccine, for example. The present invention also relates to nucleic acid molecules that are oligonucleotides capable of hybridizing, under stringent hybridization conditions, with complementary regions of a gene associated with MS containing a polymorphism of the present invention. A nucleic acid can be DNA or RNA, and single-or double-stranded. Oligonucleotides can be naturally occurring or synthetic, but are typically prepared by synthetic means. Preferred oligonucleotides include segments of DNA, or their complements. The segments are usually between 5 and 100 contiguous bases, and often range from 5, 10, 12, 15, 20, or 25 nucleotides to 10, 15, 30, 25, 20, 50 or 100 nucleotides. Nucleic acids between 5-10, 5-20, 10-20, 12-30, 15-30, 10-50, 20-50 or 20-100 bases are common. The polymorphic site can occur within any position of the segment.

Oligonucleotides can be RNA, DNA, or derivatives of either. The minimum size of such oligonucleotides is the size required for formation of a stable hybrid between an oligonucleotide and a complementary sequence on a nucleic acid molecule. The present invention relates to oligonucleotides that can be used as, for example, probes to identify nucleic acid molecules or primers to produce nucleic acid molecules. Preferred oligonucleotide probes or primers include a single base change of a polymorphism that is a focus of the present invention or the wildtype nucleotide that is located at the same position. Preferably the nucleotide of interest occupies a central position of a probe.

The nucleotide of interest may occupy a 3' position of a primer. Optionally, an array of oligonucleotides is provided, where discrete positions on the array are complementary to one or more of the provided polymorphic sequences. Such an array may comprise a series of oligonucleotides, each of which can specifically hybridize to a different polymorphism. Arrays of interest may further comprise sequences, including polymorphisms, of other genetic sequences, particularly other sequences of interest for pharmacogenetic screening. As with other human polymorphisms, the polymorphisms relating to the invention also have more general applications, such as forensic, paternity testing, linkage analysis and positional cloning.

Described herein are methods directed to diagnosing MS, clinically characterizing MS, and predicting or estimating a response to one or more therapies for the treatment of MS. The methods disclosed herein may be used to diagnose MS in a subject and to characterize the clinical course or status of MS in a subject. The methods as disclosed herein may be used to predict a response in a subject to an existing treatment for MS, or a treatment for MS that is in development or has yet to be developed. For example, the methods may be used to determine whether a patient may be more responsive to immunotherapies or to predict a response to therapy with one or more interferons or to predict a response to a treatment with one or more immunological agents. For example, the methods may be used to predict a response to a treatment with Copaxoneor to a therapy with Tysabri®.

The presence or absence of certain markers, such as clinical, biomarkers, neuroradiological, genetic and/or immunological markers, may be used to identify individuals that may be predisposed to MS, or have a greater risk or susceptibility to developing MS. Clinical, neuroradiological, genetic and/or immunological markers may be used for the stratification of MS patients according to the predicted response to one or more MS therapies. Further, clinical, neuroradiological, genetic and/or immunological markers may be used to predict the response of a subject to one or more treatments or therapies for MS. For example, the presence or absence of certain genetic markers, immunological markers or antibodies associated with MS may be used to predict the response to one or more MS therapies. The presence or absence of certain phenotypic variables, along with certain genetic markers associated with MS, may also be used to diagnose MS in a subject. Further, the presence or absence of phenotypic markers and/or genetic markers may be used to determine the clinical status of a MS patient and whether a patient is more likely to have a favorable clinical outcome with a certain MS therapy.

Te presence or absence of certain types of antibodies in MS patients may be used to predict the response in a subject to one or more treatments or therapies. Antibodies against certain molecules may be indicators or predictors of a MS disease state or a clinical response to MS therapy. Antibodies against self, or autoantibodies, may be used to identify certain populations of MS patients and/or as predictors of response to MS therapy. The presence or absence of certain antibodies may be used to measure the predisposition or risk or susceptibility to developing MS in a subject. For example, the presence or absence of anti-thyroid antibodies, or ATAbs, may be used to identify or stratify MS patients and predict the response to certain MS treatments or therapies. ATAbs against peroxidase and thyroglobulin may be used to stratify, characterize, or identify certain sub-populations of MS patients or to measure the predisposition or risk or susceptibility to developing MS in a subject, for example. As used herein, the term "susceptibility" or "susceptible" means that an individual has MS or is predisposed or at risk of developing MS.

ATAbs such as anti-thyropoetin (anti-TPO) and anti-thyroglobulin (anti-TG) may be used for the methods as described herein. Both anti-TPO and anti-TG may be used to identify certain populations of MS patients and/or as predictors of response to MS therapy and to measure the predisposition or risk or susceptibility to developing MS in a subject. An individual positive for ATAbs may be identified as a member of a population of MS patients with a predictable response to one or more MS therapies and may be identified as a member of a population of MS patients with a probability or likelihood to also have, or be predisposed to have, additional health conditions and/or disease. For example, an individual positive for one or more ATAbs may be identified as an individual more or less likely to have thyroid disease and/or other diseases or conditions. Also, an individual negative for one or more ATAbs may be identified as a sub-population of MS patients with a predictable response to MS treatments or therapies. An individual with positive or negative ATAbs may be identified or stratified as part of a population of MS patients with less severe or with more severe disease.

The presence or absence of certain clinical, neuroradiological, genetic and/or immunological markers in an individual may be associated with another condition, symptom or criteria, such as ambulatory status, neurologic status and gender to predict the presence of disease or disease outcome or the response of an individual to certain MS therapies. For example, an individual positive for certain clinical, neuroradiological, genetic and/or immunological markers and having an unassisted ambulatory status may be predicted to have a less-severe disease status. Alternatively, an individual negative for certain clinical, neuroradiological, genetic and/or immunological markers and having an unassisted ambulatory status may be predicted to have a less-severe disease status.

Antibodies, such as ATAbs, may be detected by methods known by those of skill in the art such as a chemoluminescence method. Patients may be considered positive for one or more ATAbs when ATAb levels range from approximately 25 IU/ml up to and equal to or greater than approximately 150 IU/ml. A patient with positive ATAbs may possibly have one or more ATAbs of approximately 25 IU/ml, 30 IU/ml, 35 IU/ml, 40 IU/ml, 45 IU/ml, 50 IU/ml, 55 IU/ml, 60 IU/ml, 65 IU/ml, 70 IU/ml, 75 IU/ml, 80 IU/ml, 85 IU/ml, 90 IU/ml, 95 IU/ml, 100 IU/ml, 105 IU/ml, 110 IU/ml, 115 IU/ml, 120 IU/ml, 125 IU/ml, 130 IU/ml, 135 IU/ml, 140 IU/ml, 145 IU/ml and 150 IU/ml or greater. Optionally, a patient with positive ATAbs may have one or more ATAbs from 0-3 IU/mL, 0-4 IU/mL, 0-5 IU/mL, 0-10 IU/mL, 0-15 IU/mL, and 0-20 IU/mL. In one such example, a subject may be considered positive for anti-TPO when anti-TPO levels were equal to, or greater than, a value ranging from approximately 75 IU/ml to approximately 100 IU/ml or greater. In another example, a subject may be considered positive for anti-TG when anti-TG levels are equal to, or greater than values ranging from approximately 25 IU/ml to approximately 50 IU/ml or greater. In another such example, individuals may be considered positive for ATAbs when ATAbs levels are approximately equal to or higher than 150 IU/ml, and negative for ATAbs when levels are approximately lower than 50 IU/ml.

The blood plasma levels of certain types of immunomodulating agents in an individual may be used as biomarkers to predict the response in a subject to one or more treatments or therapies. The blood plasma levels of certain cytokines may be indicators or predictors of MS in a subject or predictors of a clinical response to MS therapy. Further, the blood plasma levels of certain cytokines, in combination with the presence or absence of one or more genetic markers, may be used to measure the predisposition or risk or susceptibility to developing MS in a subject or to identify or stratify MS patients and predict the response to certain MS treatments or therapies. In one such example, the blood plasma levels of certain lymphokines, interleukins and chemokines, along with the presence of one or more genetic markers may be used to diagnose MS patients and predict the response to certain MS therapies. Cytokines, like those disclosed herein, can be proteins, peptides, and glycoproteins, including tumor necrosis factor-alpha (TNF-α), interleukin-1-beta (IL-1β), interleukin-6 (IL-6), interleukin-8 (IL-8), interleukin-4 (IL-4), interleukin-6 (IL-5), interleukin-10 (IL-10), interleukin-13 (IL-13), interferon-gamma (IFN-γ), interleukin-2 (IL-2), and interleukin-12 (IL-12). One or more cytokines may be classified as TH1 type (IFN-γ, IL-2, IL-12, etc.), TH2 type (IL-4, IL-5, IL-10, IL-13, etc.), and monokines (TNF-α, IL-1β, IL-6, IL-8, etc.). Additional immunomodulating agents, such as CD-40 ligand (CD-40L) and interleukin-2 receptor (IL-2r), may be used, in combination with the presence or absence of certain genetic markers, to diagnose MS patients and predict the response to certain MS therapies.

The blood plasma levels of one or more cytokines and immunomodulating agents, such as TNF-α, IL-1β, IL-6, IL-8, IL-4, IL-5, IL-10, IL-13, IFN-γ, IL-2, IL-12, CD-40L and IL-2r, may be used to stratify, characterize, or identify certain sub-populations of MS patients, or to measure the predisposition or risk or susceptibility to developing MS in a subject. The levels of one or more cytokines of normal healthy individuals may be compared with the cytokine levels of a subject being tested for MS, in order to measure the predisposition or risk or susceptibility to developing MS in the subject. For example, the blood plasma levels of one or more cytokines and immunomodulating agents, such as TNF-α, IL-1β, IL-6, IL-8, IL-4, IL-5, IL-10, IL-13, IFN-γ, IL-2, IL-12, CD-40L and IL-2r, may be measured in one or more normal healthy individuals and the normal cytokine levels may be compared to the cytokine levels of a subject being tested for MS, wherein cytokine levels different from the normal healthy cytokine levels are an indication of MS or the predisposition or risk or susceptibility to developing MS.

The blood plasma levels of one or more cytokines may be used to indicate the clinical disease status of a subject. For example, the levels of one or more cytokines may indicate whether a subject may be stratified or characterized as having one of four clinical courses of disease consisting of Relapsing-Remitting (RR), Primary-Progressive (PP), Secondary-Progressive (SP), and Progressive-Relapsing (PR).

Optionally, the blood plasma levels of cytokines and immunomodulating elements in an individual may range from approximately 0.0 pg/ml to 1000 pg/ml. Further, the mean value of blood plasma levels of one or more cytokines and immunomodulating agents in population may optionally range from approximately 0.1 pg/ml to approximately 500 pg/ml or greater. The mean value of blood plasma levels of one or more cytokines in a population with MS may optionally range from approximately 0.1 pg/ml up to approximately 40 pg/ml.

Optionally, the mean value of the blood plasma level of the cytokine IFN-γ in a population with MS may range from approximately 0 pg/mL to 5 pg/mL, 2 pg/ml up to 10 pg/ml, more preferably in the range from 4 pg/ml up to 8 pg/ml, and even more preferably in the range from 5 pg/ml to 6 pg/ml. Further, the mean value of the blood plasma level of the cytokine IFN-γ in a normal and healthy control population may optionally range from 0.0 pg/ml up to 2 pg/ml, more preferably in the range from 0.1 pg/ml up to 1 pg/ml, and even more preferably in the range from 0.15 pg/ml to 0.5 pg/ml. The mean value of the blood plasma level of the cytokine IFN-γ in subjects from the four clinical courses of disease may optionally be as follows: subjects with PP MS may range from 2 pg/ml up to 5 pg/ml, subjects with RR MS may range from 4 pg/ml up to 7 pg/ml, and subjects with SP MS may range from 2 pg/ml up to 5 pg/ml.

Optionally, the mean value of the blood plasma level of the cytokine IL-12 in a population with MS may range from approximately 0 pg/mL to 6 pg/mL, 0.1 pg/ml up to 15 pg/ml, more preferably in the range from 4 pg/ml up to 12 pg/ml, and even more preferably in the range from 6 pg/ml to 10 pg/ml. Further, the mean value of the blood plasma level of the cytokine IL-12 in a normal and healthy control population may optionally range from 0.0 pg/ml up to 8 pg/ml, more preferably in the range from 0.5 pg/ml up to 3 pg/ml, and even more preferably in the range from 1 pg/ml to 2 pg/ml. The mean value of the blood plasma level of the cytokine IL-12 in subjects from the four clinical courses of disease may optionally be as follows: subjects with PP MS may range from 0.1 pg/ml up to 1 pg/ml, subjects with RR MS may range from 8 pg/ml up to 12 pg/ml, and subjects with SP MS may range from 4 pg/ml up to 7 pg/ml.

Optionally, the mean value of the blood plasma level of the cytokine IL-2 in a population with MS may range from approximately 0 pg/mL to 12 pg/mL, 0.1 pg/ml up to 15 pg/ml, more preferably in the range from 4 pg/ml up to 10 pg/ml, and even more preferably in the range from 6 pg/ml to 8 pg/ml. Further, the mean value of the blood plasma level of the cytokine IL-2 in a normal and healthy control population may optionally range from 0.0 pg/ml up to 8 pg/ml, more preferably in the range from 0.5 pg/ml up to 3 pg/ml, and even more preferably in the range from 1 pg/ml to 2 pg/ml. The mean value of the blood plasma level of the cytokine IL-2 in subjects from the four clinical courses of disease may optionally be as follows: subjects with PP MS may range from 0.1 pg/ml up to 1 pg/ml, subjects with RR MS may range from 6 pg/ml up to 10 pg/ml, and subjects with SP MS may range from 1 pg/ml up to 4 pg/ml.

Optionally, the mean value of the blood plasma level of the cytokine IL-4 in a population with MS may range from approximately 0 pg/mL to 5 pg/mL, 0.1 pg/ml up to 6 pg/ml, more preferably in the range from 1 pg/ml up to 6 pg/ml, and even more preferably in the range from 2 pg/ml to 4 pg/ml. Further, the mean value of the blood plasma level of the cytokine IL-4 in a normal and healthy control population may optionally range from 0 pg/ml up to 1 pg/ml, more preferably in the range from 0.05 pg/ml up to 0.5 pg/ml, and even more preferably in the range from 0.06 pg/ml to 0.2 pg/ml. The mean value of the blood plasma level of the cytokine IL-4 in subjects from the four clinical courses of disease may optionally be as follows: subjects with PP MS may range from 0.1 pg/ml up to 1 pg/ml, subjects with RR MS may range from 1 pg/ml up to 6 pg/ml, and subjects with SP MS may range from 0.5 pg/ml up to 4 pg/ml.

Optionally, the mean value of the blood plasma level of the cytokine IL-5 in a population with MS may range from approximately 0 pg/mL to 5 pg/mL, 0.1 pg/ml up to 8 pg/ml, more preferably in the range from 1 pg/ml up to 6 pg/ml, and even more preferably in the range from 2 pg/ml to 5 pg/ml. Further, the mean value of the blood plasma level of the cytokine IL-5 in a normal and healthy control population may optionally range from 1 pg/ml up to 8 pg/ml, more preferably in the range from 2 pg/ml up to 6 pg/ml, and even more preferably in the range from 3 pg/ml to 5 pg/ml. The mean value of the blood plasma level of the cytokine IL-5 in subjects from the four clinical courses of disease may optionally be as follows: subjects with PP MS may range from 0.1 pg/ml up to 2 pg/ml, subjects with RR MS may range from 2 pg/ml up to 7 pg/ml, and subjects with SP MS may range from 1 pg/ml up to 4 pg/ml.

Optionally, the mean value of the blood plasma level of the cytokine IL-10 in a population with MS may range from approximately 0 pg/mL to 18 pg/mL, 12 pg/ml up to 25 pg/ml, more preferably in the range from 14 pg/ml up to 20 pg/ml, and even more preferably in the range from 16 pg/ml to 19 pg/ml. Further, the mean value of the blood plasma level of the cytokine IL-10 in a normal and healthy control population may optionally range from 5 pg/ml up to 12 pg/ml, more preferably in the range from 7 pg/ml up to 11 pg/ml, and even more preferably in the range from 9 pg/ml to 10 pg/ml. The mean value of the blood plasma level of the cytokine IL-10 in subjects from the four clinical courses of disease may optionally be as follows: subjects with PP MS may range from 1 pg/ml up to 4 pg/ml, subjects with RR MS may range from 12 pg/ml up to 20 pg/ml, and subjects with SP MS may range from 8 pg/ml up to 112 pg/ml.

Optionally, the mean value of the blood plasma level of the cytokine IL-13 in a population with MS may range from approximately 0 pg/mL to 5 pg/mL, 1 pg/ml up to 10 pg/ml, more preferably in the range from 2 pg/ml up to 8 pg/ml, and even more preferably in the range from 4 pg/ml to 6 pg/ml. Further, the mean value of the blood plasma level of the cytokine IL-13 in a normal and healthy control population may optionally range from 0.0 pg/ml up to 3 pg/ml, more preferably in the range from 0.2 pg/ml up to 1.5 pg/ml, and even more preferably in the range from 0.5 pg/ml to 1 pg/ml. The mean value of the blood plasma level of the cytokine IL-13 in subjects from the four clinical courses of disease may optionally be as follows: subjects with PP MS may range from 1 pg/ml up to 4 pg/ml, subjects with RR MS may range from 12 pg/ml up to 20 pg/ml, and subjects with SP MS may range from 8 pg/ml up to 112 pg/ml.

Optionally, the mean value of the blood plasma level of the cytokine IL-1β in a population with MS may range from approximately 0 pg/mL to 36 pg/mL, 25 pg/ml up to 45 pg/ml, more preferably in the range from 30 pg/ml up to 40 pg/ml, and even more preferably in the range from 33 pg/ml to 37 pg/ml. Further, the mean value of the blood plasma level of the cytokine IL-1β in a normal and healthy control population may optionally range from 5 pg/ml up to 20 pg/ml, more preferably in the range from 10 pg/ml up to 15 pg/ml, and even more preferably in the range from 12 pg/ml to 15 pg/ml. The mean value of the blood plasma level of the cytokine IL-1β in subjects from the four clinical courses of disease may optionally be as follows: subjects with PP MS may range from 5 pg/ml up to 10 pg/ml, subjects with RR MS may range from 30 pg/ml up to 40 pg/ml, and subjects with SP MS may range from 20 pg/ml up to 30 pg/ml.

Optionally, the mean value of the blood plasma level of the cytokine IL-6 in a population with MS may range from approximately 0 pg/mL to 5 pg/mL, 5 pg/ml up to 20 pg/ml, more preferably in the range from 8 pg/ml up to 16 pg/ml, and even more preferably in the range from 10 pg/ml to 14 pg/ml. Further, the mean value of the blood plasma level of the cytokine IL-6 in a normal and healthy control population may optionally range from 1 pg/ml up to 10 pg/ml, more preferably in the range from 2 pg/ml up to 6 pg/ml, and even more preferably in the range from 3 pg/ml to 5 pg/ml. The mean value of the blood plasma level of the cytokine IL-6 in subjects from the four clinical courses of disease may optionally be as follows: subjects with PP MS may range from 1 pg/ml up to 10 pg/ml, subjects with RR MS may range from 4 pg/ml up to 12 pg/ml, and subjects with SP MS may range from 1 pg/ml up to 10 pg/ml.

Optionally, the mean value of the blood plasma level of the cytokine IL-8 in a population with MS may range from approximately 0 pg/mL to 5 pg/mL, 1 pg/ml up to 8 pg/ml, more preferably in the range from 1.5 pg/ml up to 5 pg/ml, and even more preferably in the range from 2 pg/ml to 4 pg/ml. Further, the mean value of the blood plasma level of the cytokine IL-8 in a normal and healthy control population may optionally range from 1 pg/ml up to 10 pg/ml, more preferably in the range from 2 pg/ml up to 6 pg/ml, and even more preferably in the range from 3 pg/ml to 5 pg/ml. The mean value of the blood plasma level of the cytokine IL-8 in subjects from the four clinical courses of disease may optionally be as follows: subjects with PP MS may range from 1 pg/ml up to 8 pg/ml, subjects with RR MS may range from 0.5 pg/ml up to 5 pg/ml, and subjects with SP MS may range from 0.5 pg/ml up to 6 pg/ml.

Optionally, the mean value of the blood plasma level of the cytokine TNF-α in a population with MS may range from approximately 0 pg/mL to 22 pg/mL, 0.5 pg/ml up to 8 pg/ml, more preferably in the range from 1 pg/ml up to 5 pg/ml, and even more preferably in the range from 2 pg/ml to 4 pg/ml. Further, the mean value of the blood plasma level of the cytokine TNF-α in a normal and healthy control population may optionally range from 0.1 pg/ml up to 5 pg/ml, more preferably in the range from 0.5 pg/ml up to 4 pg/ml, and even more preferably in the range from 1 pg/ml to 2 pg/ml. The mean value of the blood plasma level of the cytokine TNF-α in subjects from the four clinical courses of disease may optionally be as follows: subjects with PP MS may range from 0.0 pg/ml up to 2 pg/ml, subjects with RR MS may range from 0.5 pg/ml up to 5 pg/ml, and subjects with SP MS may range from 0.5 pg/ml up to 6 pg/ml.

The blood plasma levels of one or more immunomodulating agents, such as CD-40L and IL-2r, may be used to stratify, characterize, or identify certain sub-populations of MS patients or to measure the predisposition or risk or susceptibility to developing MS in a subject. The mean value of blood plasma levels of one or more immunomodulating agents in a subject with MS may optionally range from approximately 50 pg/ml up to approximately 500 pg/ml. For example, the mean value of the blood plasma level of CD-40L in a population with MS may optionally range from approximately 20 pg/ml up to 100 pg/ml, more preferably in the range from 40 pg/ml up to 80 pg/ml, and even more preferably in the range from 50 pg/ml to 70 pg/ml. Optionally, the blood plasma levels of CD-40L may range from 0 pg/mL to less than 244 pg/mL. Further, the mean value of the blood plasma level of CD-40L in a normal and healthy control population may optionally range from 60 pg/ml up to 100 pg/ml, more preferably in the range from 70 pg/ml up to 90 pg/ml, and even more preferably in the range from 75 pg/ml to 85 pg/ml. The mean value of the blood plasma level of CD-40L in subjects from the four clinical courses of disease may optionally be as follows: subjects with PP MS may range from 100 pg/ml up to 130 pg/ml, subjects with RR MS may range from 45 pg/ml up to 65 pg/ml, and subjects with SP MS may range from 40 pg/ml up to 60 pg/ml.

Optionally, the mean value of the blood plasma level of IL-2r in a population with MS may range from approximately 0 pg/mL to 1033 pg/mL, 350 pg/ml up to 500 pg/ml, more preferably in the range from 430 pg/ml up to 480 pg/ml, and even more preferably in the range from 450 pg/ml to 470 pg/ml. Further, the mean value of the blood plasma level of IL-2r in a normal and healthy control population may optionally range from 400 pg/ml up to 600 pg/ml, more preferably in the range from 450 pg/ml up to 550 pg/ml, and even more preferably in the range from 475 pg/ml to 525 pg/ml. The mean value of the blood plasma level of IL-2r in subjects from the four clinical courses of disease may optionally be as follows: subjects with PP MS may range from 400 pg/ml up to 500 pg/ml, subjects with RR MS may range from 400 pg/ml up to 500 pg/ml, and subjects with SP MS may range from 450 pg/ml up to 550 pg/ml.

The teachings disclosed herein provide a collection of polymorphisms in genes or chromosomal regions associated with MS. Detection of polymorphisms is useful in designing and performing diagnostic assays for evaluation of genetic risks or susceptibility for MS and other related conditions. Analysis of polymorphisms is also useful in designing prophylactic and therapeutic regimes customized to MS treatments. Detection of polymorphisms is also useful for conducting clinical trials of drugs for treatment of MS. The teachings disclosed herein also provide methods and compositions for clinical screening and diagnosis of MS in a subject and for identifying patients most likely to respond to a particular therapeutic treatment, for monitoring the results of MS therapy, and for drug screening and drug development.

Polymorphism refers to the occurrence of two or more genetically determined alternative sequences or alleles in a population. A polymorphic genetic marker or site is the locus at which divergence occurs. In one scenario, genetic markers have at least two alleles, each occurring at a frequency of greater than 1%, and more preferably greater than 10% or 20% of a selected population. A polymorphic locus may be as small as one base pair.

Polymorphic genetic markers may include single nucleotide polymorphisms (SNP), restriction fragment length polymorphisms, variable number of tandem repeats (VNTRs), hypervariable regions, minisatellites, dinucleotide repeats, trinucleotide repeats, tetranucleotide repeats, simple sequence repeats, and insertion elements.

A single nucleotide polymorphism (SNP) occurs at a polymorphic site occupied by a single nucleotide, which is the site of variation between allelic sequences. A single nucleotide polymorphism may arise due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele.

The presence or absence of one or more genetic markers may be predictive of whether an individual is at risk or susceptibly to MS. One or more genetic markers may optionally be associated with a disease phenotype by the use of a genome wide association study (GWAS). As generally know by those of skill in the art, a GWAS is an examination of genetic polymorphism across a given genome, designed to identify genetic associations with a trait or phenotype of interest, such as MS. If genetic polymorphisms are more frequent in people with MS, the variations are said to be "associated" with MS. The polymorphisms associated with MS may either directly cause the disease phenotype or they may be in linkage disequilibrium with nearby genetic mutations that influence the individual variation in the disease phenotype. Linkage disequilibrium, as used herein, is the non-random association of alleles at two or more loci.

A GWAS may optionally be conducted using a DNA microarray as generally known in the art. Array-based detection can be performed to detect genetic polymorphisms. Commercially available arrays, e.g., from Affymetrix, Inc. (Santa Clara, Calif.) or other manufacturers may be used to detect polymorphisms. Reviews regarding the operation of nucleic acid arrays include Sapolsky et al. (1999) "High-throughput polymorphism screening and genotyping with high-density oligonucleotide arrays." Genetic Analysis: Biomolecular Engineering 14:187-192; Lockhart (1998) "Mutant yeast on drugs" Nature Medicine 4:1235-1236; Fodor (1997) "Genes, Chips and the Human Genome." FASEB Journal 11:A879; Fodor (1997) "Massively Parallel Genomics." Science 277: 393-395; and Chee et al. (1996) "Accessing Genetic Information with High-Density DNA Arrays." Science 274:610-614.

As generally known in the art, a variety of probe arrays can be used for detection of polymorphisms that can be correlated to the phenotypes of interest. For example, DNA probe array chips or larger DNA probe array wafers (from which individual chips would otherwise be obtained by breaking up the wafer) may be used. DNA probe array wafers may optionally comprise glass wafers on which high density arrays of DNA probes (short segments of DNA) have been placed. Each of these wafers can hold, for example, millions of DNA probes that are used to recognize sample DNA sequences (e.g., from individuals or populations that may comprise polymorphisms of interest). The recognition of sample DNA by the set of DNA probes on the glass wafer takes place through DNA hybridization. When a DNA sample hybridizes with an array of DNA probes, the sample binds to those probes that are complementary to the sample DNA sequence. By evaluating to which probes the sample DNA for an individual hybridizes more strongly, it is possible to determine whether a known sequence of nucleic acid is present or not in the sample, thereby determining whether a polymorphism found in the nucleic acid is present.

The use of DNA probe arrays to obtain allele information typically involves the following general steps: design and manufacture of DNA probe arrays, preparation of the sample, hybridization of sample DNA to the array, detection of hybridization events and data analysis to determine sequence. Wafers may be manufactured using a process adapted from semiconductor manufacturing to achieve cost effectiveness and high quality, and are available, e.g., from Affymetrix, Inc. of Santa Clara, Calif.

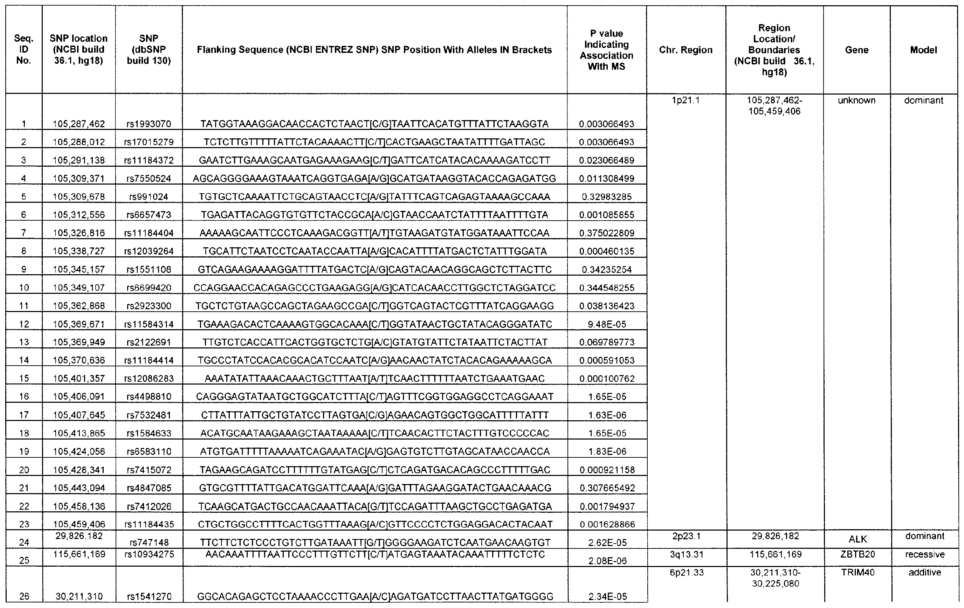

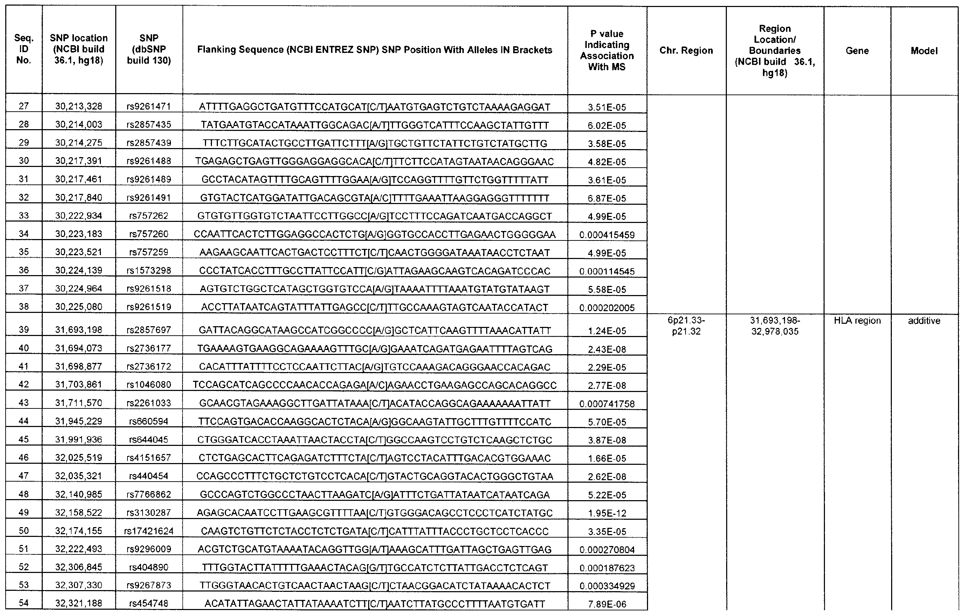

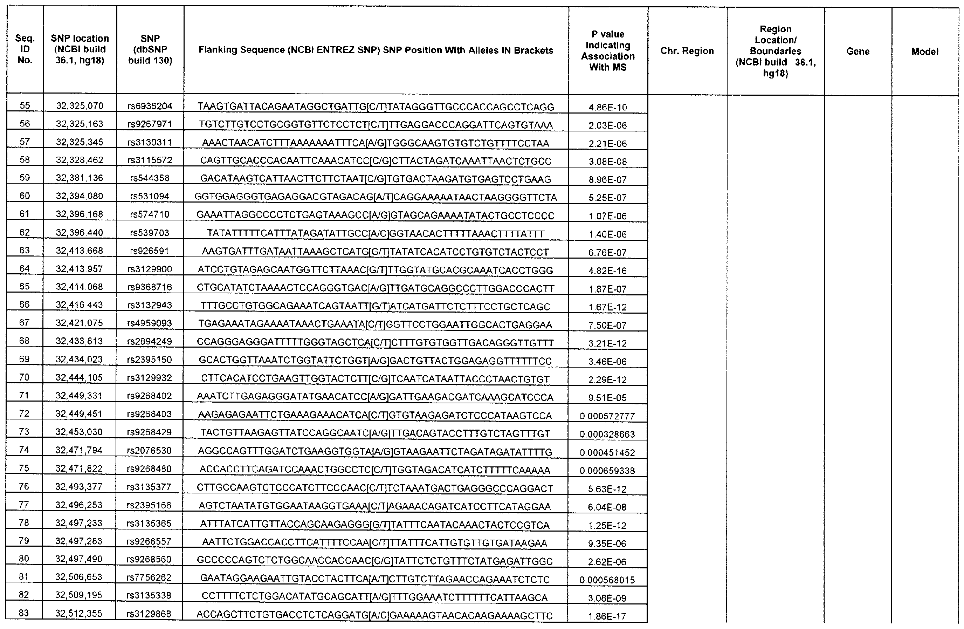

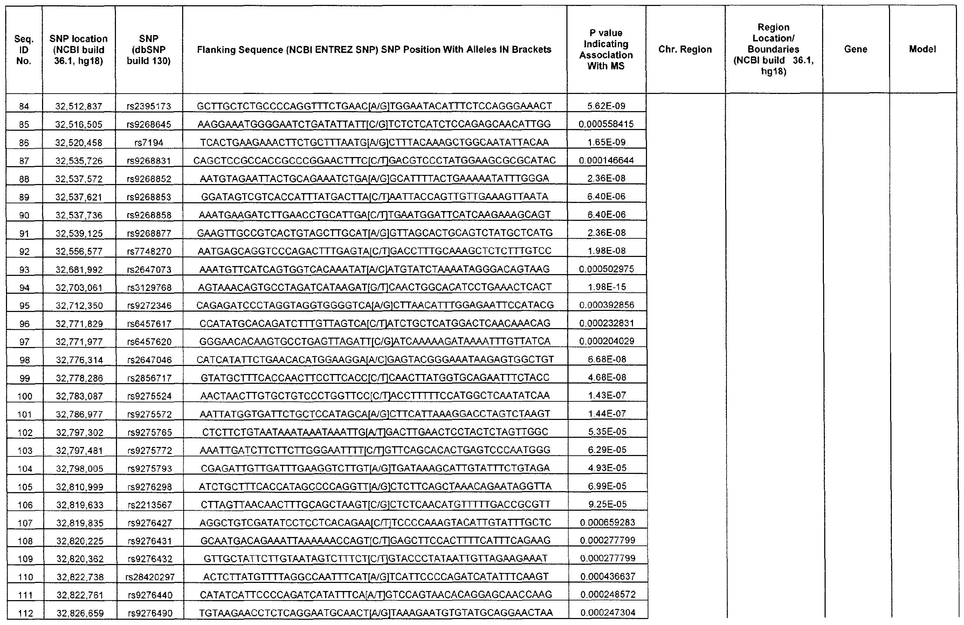

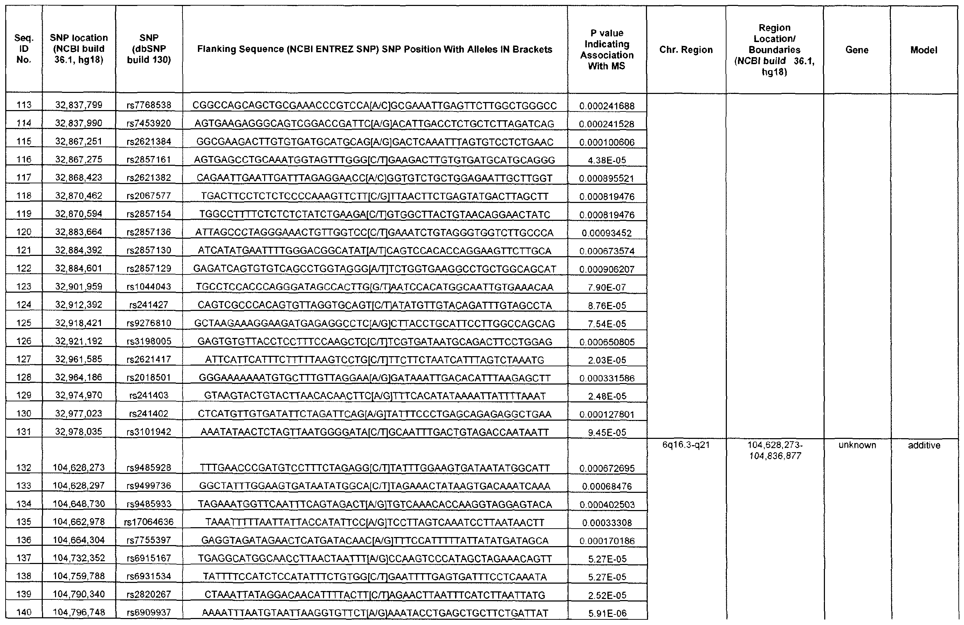

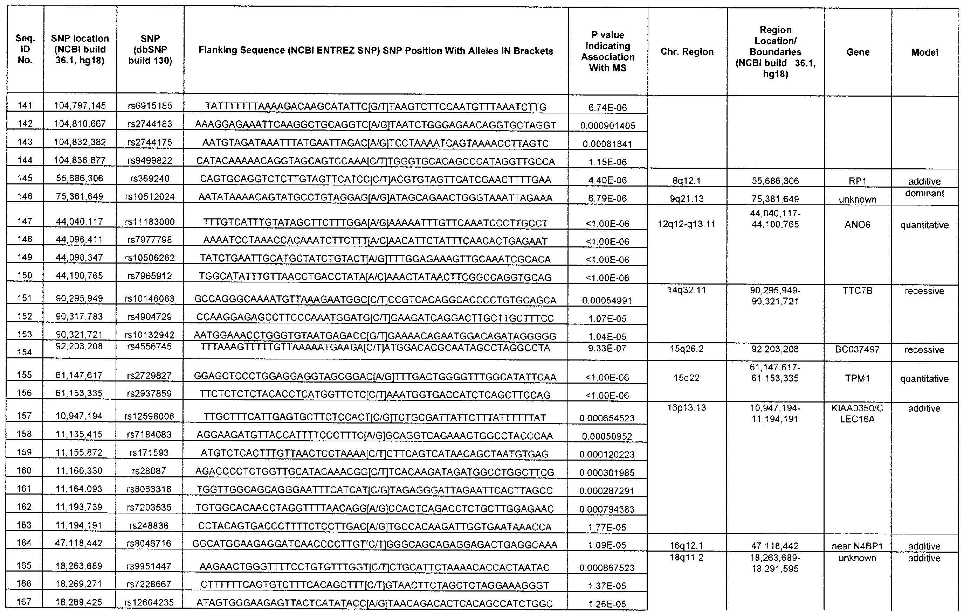

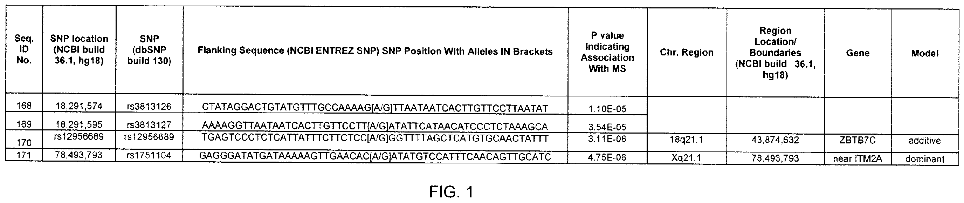

Genetic markers associated with MS and used to diagnose a predisposition or increased risk or susceptibility to MS, or a response to a MS therapeutic, may include one or more SNPs. As disclosed herein, a SNP may be identified by its name or by location within a particular sequence. The SNPs identified in the SEQ. ID. NOS. of FIG. 1 are indicated by brackets. For example, the SNP "[C/G]" in SEQ. ID. NO.: 1 of FIG. 1 indicates that the nucleotide base (or the allele) at that position in the sequence may be either cytosine or guanine. The nucleotides flanking the SNP for each SEQ. ID. NO. in FIG. 1 are the flanking sequences which may be used to identify the location of the SNP in the genome.

As used herein, the nucleotide sequences disclosed herein encompass the complements of said nucleotide sequences. In addition, as used herein, the term "SNP" encompasses any allele among a set of alleles. The term "allele" refers to a specific nucleotide among a selection of nucleotides defining a SNP.

The SNPs as provided herein may include isolated polynucleotides comprising a SNP located within a sequence selected from the group consisting of sequences identified by SEQ. ID. NOS.: 1-171 and the complements of sequences identified by SEQ. ID. NOS.: 1-171; wherein the presence of a particular allele of a SNP (a particular nucleotide base) is indicative of a propensity to develop MS or otherwise may be used to identify a subject with MS. In one example, the polynucleotide is selected from the group consisting of sequences identified by SEQ. ID. NOS.: 1-171 and the complements of sequences identified by SEQ. ID. NOS.: 1-171. In another example, the polynucleotide comprises at least a portion of a sequence selected from the group consisting of sequences identified by SEQ. ID. NOS.: 1-171 and the complements of sequences identified by SEQ. ID. NOS.: 1-171.

Polymorphisms associated with MS and used to diagnose a predisposition or increased risk of MS, or a response to a MS therapeutic, may include one or more loci located in a particular region of a chromosome. Polymorphisms associated with MS may optionally be selected from one or more of the chromosal regions comprising 1p21.1, 2p23.2-p23.1 (ALK gene), 3q13.31 (ZBTB20 gene), 6p21.33-p21.32 (HLA region), 6p21.33 (TRIM40 gene), 6q16.3-q21, 8q12.1 (RP1 gene), 9q21.13, 12q12-q13.11 (ANO6 gene), 14q32.11 (TTC7B gene), 15q26.2 (BC037497 gene), 15q22 (TPM1 gene), 16p13.13 (KIAA0350/CLEC16A genes), 16q12.1, 18q11.2, 18q21.1 (ZBTB7C gene), and Xq21.1 (near ITM2A gene).

The methods disclosed herein may comprise assaying the presence of one or more polymorphisms in an individual which may include methods generally known in the art. Methods for assaying a genetic polymorphism in an individual may include assaying an individual for the presence or absence of a SNP associated with MS using one or more genotyping assays such as a SNP array, PCR-based SNP genotyping, DNA hybridization, fluorescence microscopy, and other methods known by those of skill in the art. Furthermore, methods for assaying the presence or absence of one or more SNP markers associated with MS may include providing a nucleotide sample from an individual and assaying the nucleotide sample for the presence or absence of one or more SNP markers. The nucleotide sample may optionally include, e.g., a biological fluid or tissue. Examples of biological fluids include, e.g., whole blood, serum, plasma, cerebrospinal fluid, urine, tears or saliva. Examples of tissue include, e.g., connective tissue, muscle tissue, nervous tissue, epithelial tissue, and combinations thereof.

Methods for identifying subjects with MS or individuals predisposed or at risk of developing MS are provided. Methods for predicting the response to a MS treatment or therapy are also discussed. In one embodiment, the method comprises the steps of obtaining a sample from a subject, measuring the blood plasma levels of one or more biomarkers, such as cytokines, immunomodulating agents and antibodies as disclosed herein, and detecting one or more alleles, polymorphisms, genetic markers associated with MS, wherein a certain blood plasma level of a biomarker and the identification of one or more genetic markers associated with MS indicates subjects with MS or individuals predisposed or at risk of developing MS. For example, a subject may provide a test sample that is tested for the blood plasma levels of one or more cytokines or immunomodulating agents taught herein as well as tested for the presence or absence of one or more genetic markers. The results of the test, showing the cytokine levels and the presence or absence of one or more genetic markers, may then be used to indicate certain sub-populations of MS patients, or to measure the predisposition or risk of developing MS in a subject or predict the response to MS therapies. The test sample can be, e.g., a biological fluid or tissue. Examples of biological fluids include, e.g., whole blood, serum, plasma, cerebrospinal fluid, urine, tears or saliva. Examples of tissue include, e.g., connective tissue, muscle tissue, nervous tissue, epithelial tissue, and combinations thereof.

Optionally, the response to a MS therapy may be predicted, or an individual with MS or a predisposition or risk of developing MS is identified, by the steps comprising: collecting a test sample from a subject, measuring the blood plasma level of one or more of TNF-α, IL-1β, IL-6, IL-8, IL-4, IL-5, IL-10, IL-13, IFN-γ, IL-2, IL-12, CD-40L, IL-2r, detecting the presence of one or more genetic markers associated with MS, wherein the level of TNF-α, IL-1β, IL-6, IL-8, IL-4, IL-5, IL-10, IL-13, IFN-γ, IL-2, IL-12, CD-40L, or IL-2r and the presence of the one or more genetic markers associated with MS identifies an individual with MS or a predisposition or risk of developing MS. In another embodiment, the response to a MS therapy may be predicted or an individual with MS or a predisposition or risk of developing MS is identified by the steps comprising: collecting a test sample from a subject, measuring the presence of anti-TPO antibodies or anti-TG antibodies, or a combination thereof, detecting the presence of one or more genetic markers associated with MS, wherein the presence of anti-TPO antibodies or anti-TG antibodies and the presence of the one or more genetic markers associated with MS identifies an individual with MS or a predisposition or risk of developing MS or predicts the response for a MS therapy.

According to an aspect of the present invention there is provided a method of determining the susceptibility to multiple sclerosis (MS) in an individual, the method comprising:
providing a sample obtained from an individual in need of testing for the susceptibility of MS;
assaying the sample for the presence in the individual of at least one of: a G allele SNP at position 27 of SEQ ID NO.: 147, an A allele SNP at position 27 of SEQ ID NO.: 148, a G allele SNP at position 27 of SEQ ID NO.: 149, and an A allele SNP at position 27 of SEQ ID NO.: 150;
wherein the presence in the individual of at least one of a G allele SNP at position 27 of SEQ ID NO.: 147, an A allele SNP at position 27 of SEQ ID NO.: 148, a G allele SNP at position 27 of SEQ ID NO.: 149, and an A allele SNP at position 27 of SEQ ID NO.: 150 is indicative that the individual is susceptible to MS.

The method may further comprise assaying for the presence or absence in the individual of at least one further biomarker associated with MS, wherein the sample is a blood plasma sample, and wherein the presence in the individual of at least one SNP as recited in claim 1 and the presence in the individual of the at least one biomarker associated with MS is indicative that the individual is susceptible to MS. A further biomarker or biomarkers may be selected from the group consisting of at least one anti-thyroid antibody, at least one cytokine, and at least one immunomodulating agent, or combinations thereof.

Cytokine and immunomodulating agent biomarkers may include at least one of TNF-α, IL-1β, IL-6, IL-8, IL-4, IL-5, IL-10, IL-13, IFN-γ, IL-2, IL-12, CD-40L and IL-2r, preferably IL-1β, I L-2, I L-6, TNF-α, and I L-4.

Assaying for the presence of the at least one further biomarker in the individual may comprise assaying the blood plasma level of at least one of TNF-α, IL-1β, IL-6, IL-8, IL-4, IL-5, IL-10, IL-13, IFN-γ, IL-2, IL-12, CD-40L and IL-2r, at a level approximately greater than the mean blood plasma level of the at least one biomarker in a normal healthy control population.

The method may further comprise assaying the sample for the presence of at least one of: a G allele SNP at position 27 of SEQ ID NO.: 155, and a C allele SNP at position 27 of SEQ ID NO.: 156, and wherein the at least one further biomarker is IL-4, wherein the presence in an individual of a G allele at position 27 of SEQ ID NO.: 155 and/or a C allele at position 27 of SEQ ID.: 156 is indicative that the individual is susceptible to MS.

A further biomarker may be IL-1β and the method may comprise detecting it at a blood plasma level greater than the mean blood plasma level of IL-1β in a normal healthy control population, for example approximately 35 pg/mL.

A further biomarker may be IL-2 and the method may comprise detecting it at a blood plasma level ranging from greater than the mean blood plasma level of IL-2 in a normal healthy control population, for example approximately 7 pg/mL.

A further biomarker may be IL-6 and the method may comprise detecting it at a blood plasma level greater than the mean blood plasma level of IL-6 in a normal healthy control population, for example approximately 12 pg/mL.

A further biomarker may be TNF-α and the method may comprise detecting it at a blood plasma level greater than the mean blood plasma level of TNF-α in a normal healthy control population, for example approximately 2 pg/mL.

A further biomarker may be IL-4 and the method may comprise detecting it at a blood plasma level greater than the mean blood plasma level of IL-4 in a normal healthy control population, for example approximately 2 pg/mL.

According to another aspect of the present invention there is provided an in vitro diagnostic kit for indicating the susceptibility to multiple sclerosis (MS) in an individual using a method according to claim 1, wherein the kit comprises:
a product for the specific detection of
   the presence in the individual of at least one of: a G allele SNP at position 27 of SEQ ID NO.: 147, an A allele SNP at position 27 of SEQ ID NO.: 148, a G allele SNP at position 27 of SEQ ID NO.: 149, and an A allele SNP at position 27 of SEQ ID NO.: 150; and a product for the specific detection of
   the presence in the individual of at least one further biomarker associated with MS;
wherein the at least one further biomarker is at least one of TNF-α, IL-1β, IL-6, IL-8, IL-4, IL-5, IL-10, IL-13, IFN-γ, IL-2, IL-12, CD-40L, IL-2r, anti-thyroid peroxidise (anti-TPO) antibody and anti-thyroglobulin (anti-TG) antibody.

The following examples are given to illustrate various embodiments which have been made with the present invention. It is to be understood that the following examples are provided by way of illustration and nothing therein should be taken as a limitation upon the overall scope of the many embodiments which can be prepared in accordance with the present invention.

### Examples

The Examples that follow are offered for illustrative purposes only and are not intended to limit the scope of the compositions and methods described herein in any way. In each instance, unless otherwise specified, standard materials and methods were used in carrying out the work described in the Examples provided.

The practice of the present invention employs, unless otherwise indicated, conventional techniques of chemistry, molecular biology, microbiology, recombinant DNA, genetics, immunology, cell biology, cell culture and transgenic biology, which are within the skill of the art (See, e.g., Maniatis, T., et al. (1982) Molecular Cloning: A Laboratory Manual (Cold Spring Harbor Laboratory, Cold Spring Harbor, N.Y.); Sambrook, J., et al. (1989) Molecular Cloning: A Laboratory Manual, 2nd Ed. (Cold Spring Harbor Laboratory, Cold Spring Harbor, N.Y.); Ausubel, F. M., et al. (1992) Current Protocols in Molecular Biology, (J. Wiley and Sons, NY); Glover, D. (1985) DNA Cloning, I and II (Oxford Press); Anand, R. (1992) Techniques for the Analysis of Complex Genomes, (Academic Press); Guthrie, G. and Fink, G. R. (1991) Guide to Yeast Genetics and Molecular Biology (Academic Press); Harlow and Lane (1988) Antibodies: A Laboratory Manual (Cold Spring Harbor Laboratory, Cold Spring Harbor, N.Y.); Jakoby, W. B. and Pastan, I. H. (eds.) (1979) Cell Culture. Methods in Enzymology, Vol. 58 (Academic Press, Inc., Harcourt Brace Jovanovich (NY); Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Hogan et al. (eds) (1994) Manipulating the Mouse Embryo. A Laboratory Manual, 2nd Edition, Cold Spring Harbor Laboratory Press, Cold Spring Harbor, N.Y. A general discussion of techniques and materials for human gene mapping, including mapping of human chromosome 1, is provided, e.g., in White and Lalouel (1988) Ann. Rev. Genet. 22:259 279.

### Example 1

Multiple sclerosis is a complex autoimmune disease which may be associated with other autoimmune diseases such as autoimmune thyroid disorders. Thyroid dysfunction is commonly reported in the clinic, consisting of a reported incidence of hypo or hyperthyroidism. Often diagnostic testing supporting an autoimmune pathogenesis for these thyroid conditions is absent.

In a demographic survey of clinically definite MS (CDMS) patients at the University of Utah, there did not appear to be an over representation of autoimmune diseases except for those of the thyroid including Hashimoto's Thyroiditis and Graves Disease. This was in distinction to other autoimmune diseases which are occasionally observed in conjunction with MS, such as systemic lupus erythematousis, rheumatoid arthritis, Sjogren's disease, inflammatory bowel disorders, anti phospholipid antibody syndrome, B12 deficiency and diabetis type I. To obtain a better understanding of autoimmunity in the CDMS patients, the levels of anti-thyroid antibodies (ATAbs) were determined, as well as the association of ATAbs with a clinical diagnosis of autoimmune thyroid disease, duration of MS, gender and ambulatory status.

### Methods

### Patient Population:

The patient population comprised 640 CDMS Patients recruited from the Multiple Sclerosis Clinic at the University of Utah. McDonald Criteria were used to for determination of CDMS. The disease duration ranged from 0 to >30 years. The patients' ambulatory status was characterized as unassisted, assisted or wheelchair dependent. The patients consisted of 485 Female and 155 Males (F/M=3.15). Anti-thyroid Antibody Analyses:
A total of 640 CDMS patients were tested for the presence of anti-thyroid antibodies (ATAbs), including anti-thyropoetin (anti-TPO) and anti-thyroglobulin (anti-TG) antibodies. Positive results for ATAbs means positive for either anti-TPO, anti-TG, or both. Antibodies to the thyroid antigens were measured by chemiluminescent microparticle immunoassay (Abbot, Diagnostics Division). The levels of > 100 IU for anti-TPO and > 50 IU for anti-TG antibodies were considered positive as described in the literature (Table 1). More particularly, this study utilized criteria and methods as discussed by Polman C, et al. Interferon beta 1 b does not induce autoantibodies, Neurology 2005; 64:996-1000; Munteis, E, et al. Prevalence of autoimmune thyroid disorders in a Spanish multiple sclerosis cohort, European Journal of Neurology 2007; 14: 1048-1052; and Ramagopalan S, et al. Autoimmue disease in families with multiple sclerosis: a population based study, Lancet Neurology; 6, 575-576.

**TABLE 1**

| **Antibody** | **F** | **M** | **P Value** |
|---|---|---|---|
| ATAbs+ | 64 | 8 | P < 0.006 |
| Anti-TPO+ | 46 | 8 | P < 0.1 |
| Anti-TG+ | 38 | 4 | P < 0.03 |
| Both AB+ | 20 | 4 | NS |

### Results

### ATAbs Criteria Verified:

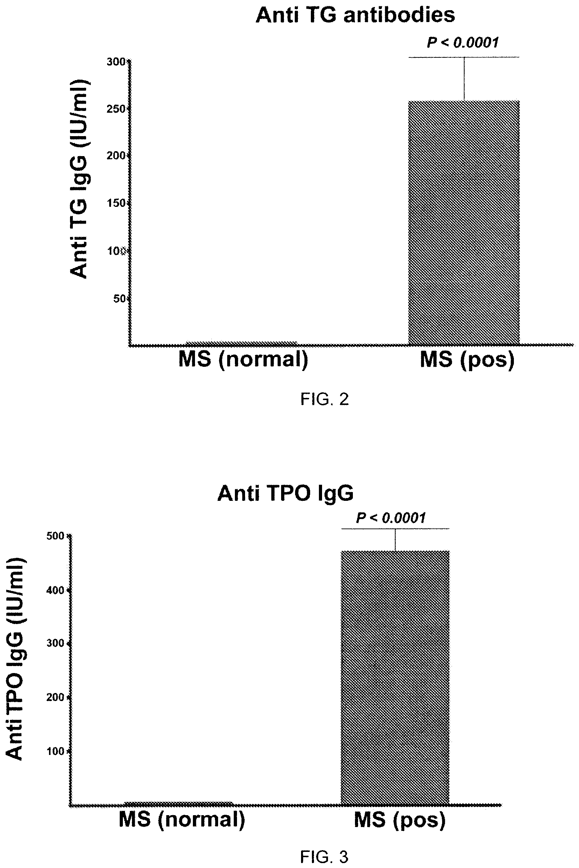

To determine the reliability of the criteria the difference in means between the ATAb positive and negative patients were tested. As shown in FIG. 2 and FIG. 3, the validity of the criteria was verified for both anti-TPO and anti-TG antibodies (p<0.0001).

### ATAbs in CDMS Patients:

For the CDMS patients, 72/640 (11.25%) were positive for ATAbs, with 64 Female and 8 Male (F/M=8.0). Duration of MS was not a factor in these patients Diagnosed Thyroid Disease in ATAbs-positive CDMS Patients:

For those CDMS patients positive for ATAbs, 31/72 (43%) were diagnosed with Hashimoto's Thyroiditis or Graves disease (30 female, 1 male).

### Gender and ATAbs:

As shown in Table 2, ATAbs and anti-TG antibodies were significantly more common in females. Anti-TPO antibodies alone did not reach significance.

**TABLE 2**

| **Antibody** | **Polman Criteria for Positive** |
|---|---|
| Anti-TPO | ≥ 100 IU |
| Anti-TG | ≥ 50 IU |
| ATAbs | Positive for either TPO, TG or both |

| | |
|---|---|
| Fisher's exact test two-sided p value | |

### Ambulation and ATAbs:

To evaluate disease severity and ATAbs, ambulatory status was observed. When patients with unassisted ambulation were compared to those either requiring assistance or a wheel chair the data for anti-TPO suggested a possible association of these antibodies with higher level of ambulation (Fisher's exact test one sided p value <0.05).

### Conclusion

ATAbs were associated with gender bias (F/M ratio = 8.0) and a significant incidence of clinically diagnosed thyroid disease (Hashimoto's thyroiditis or Graves Disease 43%) in which females exceed males by 30:1. Anti-TPO antibodies may be associated with less severe disease as indicated by ambulatory status. Patients positive for ATAbs represent a sub-population of MS patients with more generalized autoimmunity. Thus, one aspect of ATAbs might be their use in stratification of MS patients during the process of determining genes involved in multiple sclerosis. As the presence of ATAbs can be used to distinguish MS patients with a higher propensity for autoimmunity, specific pathways involved in the immune response and autoimmunity may be discovered, leading to optimal selection of existing treatments and the development of new therapies. The presence or absence of such antibodies (and other phenotypic variables) may be integrated with genetic variations associated with disease to provide improved MS diagnosis.

These data suggest a better ambulatory outcome in patients with ATAbs. These antibodies differentiate a population of MS patients that have significant autoantibodies involved in immunologic and inflammatory pathways that may be targeted by current therapies, therapies in development and future treatments that may be discovered. The development of ATAbs may be related to alterations in T cell regulation and T regulatory cells (Tregs). Treatments that alter T regulatory function should therefore be particularly effective in the subpopulation of CDMS patients designated by abnormal levels of ATAbs.

Thus, the presence of ATAbs in CDMS patients reveals that such patients are predicted to be more responsive to immune therapies. The presence of ATAbs is also associated with a better prognosis.

### Example 2

### Population Based SNP Association Study

### Case/Control Genome Wide Analysis

MS cases and controls were genotyped on the Affymetrix 6.0 array according to standard procedures and protocols (Affymetrix, Inc., Santa Clara, CA). The samples were analyzed in 4 batches, totaling 1248 individuals, with 500 cases and 748 controls, all drawn from a Utah population. The sample data files generated from the Affymetrix 6.0 array were analyzed with the Golden Helix SNP & Variation Suite (Golden Helix, Inc., Bozeman, MT). Dominant and recessive correlation/trend association tests were performed on the autosomal markers.

### Quantative Trait Loci Association Testing

A total of 15 phenotypes were scored for each of the 500 MS case individuals. The phenotypes scored were anti-TPO antibody (0-3.9 IU/mL), anti-TG antibody (0-14.4 IU/mL), CD-40L (<244 pg/mL), IL-2r (0-1033 pg/mL), TNF-α (0-22 pg/mL), IL-1β (0-36 pg/mL), IL-6 (0-5 pg/mL), IL-8 (0-5 pg/mL), IL-4 (0-5 pg/mL), IL-5 (0-5 pg/mL), IL-10 (0-18 pg/mL), IL-13 (0-5 pg/mL), IFN-γ (0-5 pg/mL), IL-2 (0-12 pg/mL), and IL-12(0-6pg/mL).

### Results

FIG. 1 shows the significant chromosome regions and SNPs discovered using the case-control association analysis and the quantitative trait loci (QTL) association testing.

For the QTL analysis, four SNPs had at least one phenotype with a -log10 p-value of 6 in the Chr12q12 consisting of rs1118300 (SEQ ID NO: 147), rs7977798 (SEQ ID NO: 148), rs1050626 (SEQ ID NO: 149), and rs7965912 (SEQ ID NO: 150). The phenotypes that had the p-values that were at most 1e-06 were IL-1β (all four SNP markers), IL-2 (last two markers), IL-6 (all but second marker), TNF-α (last marker only). These markers are in the ANO6 gene.

Two SNPs, rs4556745 (SEQ ID NO: 154) and rs2729827 (SEQ ID NO: 155) in the TPM1 gene, had at least one phenotype with a -log10 p-value of 6 in the Chr15q22.2 region. The phenotype that had the p-values that were at most 1e-06 was IL-4 for both of the chromosome 15 SNP markers.

### Example 3

### MS: Family Linkage analysis

A family linkage analysis was performed using MS families consisting of 234 individuals from 55 families with a high incidence of MS. These families encompassed a total of 102 unaffected subjects and 132 definitive MS affected members, with 5 of these families having ≥4 affected individuals and one family having 10 MS patients.

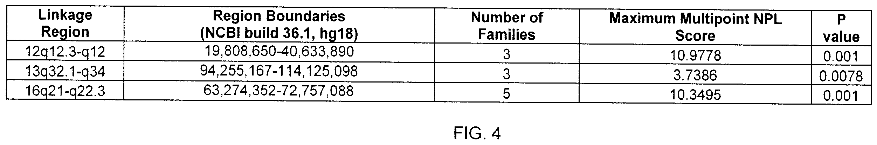

Multipoint linkage analysis using GeneHunter (easyLinkage-Plus, Lindner and Hoffmann, 21, 3565-3567, *Bioinformatics* 2005) revealed three major peaks (FIG. 4). Each locus was identified in at least three families. In addition, one region on chromosome 12 had been previously reported by Vitale et al. (11, 295-300, *Human Molecular Genetics,* 2002) who reported that gene ST8SIA1 within the chromosome 12 region 12q12.3-q12 contains SNPs associated with MS, thereby adding more significance to these findings disclosed herein. For example, the region on chromosome 12, 12q12.3-q12, contains the FGD4 gene known to cause peripheral demyelinating disease, CMT4H. Furthermore, the region on chromosome 16, 16q21-q22.3, contains TRADD. This gene product interacts with TNFRSF1A gene product which is a known risk factor of MS.

### Example 4

### Biomarkers and MS by disease state

Blood plasma levels of biomarkers, including cytokine biomarkers and immunomodulating biomarkers, were compared among a normal healthy control sample and an MS patient sample. The cytokine biomarkers assayed were TNF-α, IL-1β, IL-6, IL-8, IL-4, IL-5, IL-10, IL-13, IFN-γ, IL-2, IL-12. The immunomodulating biomarkers assayed were CD-40L and IL-2r.

### Methods

Monoclonal antibodies that bind the biomarkers were prepared for use as capture antibodies to assay the biomarkers in the sample populations. The biomarkers were assayed by methods generally known in the art and, for example, described by H.R. Hill, T.B. Martins, Methods 38 (2006) 312-316. More particularly, the monoclonal antibodies were diluted in coupling buffer (50 mM 2-[*N-*morpholino]ethanesulfonic acid (Mes) (Sigma, St. Louis, MO), pH 5.0) to concentrations ranging 50-100 µg/mL and covalently coupled to carboxylated Luminex microspheres (Luminex Corporation, Austin, TX.) using a two-step carbodiimide reaction. Internal controls for the multiplex assay were made by coating individual bead sets with normal mouse and rat IgG (50 µg/mL; Sigma, St. Louis, MO) or pooled normal mouse serum (100 µg/mL; Sigma). The carboxylated microspheres were activated for 20 min at a concentration of 6.25 × 10⁶/mL in PBS, pH 6.1, with 5 mg/mL of 1-ethyl-3-(3-dimethylaminopropyl) carbodiimide and *N-*hydroxy-sulfo-sulfosuccinimide (Pierce-Endogen, Rockford, IL). Activated microspheres were then washed with coupling buffer, and incubated with the previously described monoclonal antibodies for 2 h at room temperature on a rocker. The coupled microspheres were then washed twice with blocking-storage buffer (PBS, 0.1 % BSA, 0.02% Tween, 0.05% azide, pH 7.4) and resuspended in 1 mL of blocking-storage buffer. The microspheres were then incubated for 30 min on a rocker to permit blocking of the unreacted sites and stored at 4 °C in PBS. All activation, centrifugation and incubations were carried out in the dark, since the fluorescent dyes used to identify the beads are light sensitive.

A standard curve for each biomarker was developed by mixing known quantities of recombinant human cytokines IL-2, IL-4, IL-6, IL-10, IL-12, and IFN-γ in RPMI-1640 media for culture supernatant assays. For the serum/plasma assay, four additional cytokines/receptors, IL-2 receptor, TNF-α, IL-8, and IL-1β, were included. The serum/plasma sample diluent contained 10% v/v fetal bovine serum (FBS) and 5% v/v mouse serum, and 2.5% v/v rat serum (Sigma) diluted in PBST (PBS, 0.02% Tween 20, pH 7.4) containing 0.05% Proclin (Sigma). Typical dynamic ranges were from 0 to 10,000 pg/mL and were represented by an seven-point standard curve. Standards were run in duplicate along with three controls containing high, medium, and low concentrations of cytokines. The microspheres were mixed at a concentration of 5000 microspheres of each monoclonal coupled capture antibody per reaction. Fifty microliters of the combined microsphere mixture was added to either 100 µL of tissue culture supernatant or 150 µL of diluted serum. The cytokine standards, controls, tissue culture supernatants, or serum and coated microspheres are then incubated for 20 min (culture supernatants) or 2 h (serum/plasma) at room temperature in 96-well filter microtiter plates (Millipore, Bedford, MA) on a rotator. Microspheres were then washed three times with 200 µL of PBST and vacuum filtered. This was followed by the addition of 100 µL of biotinylated poly or monoclonal antibodies specific for each of the biomarkers with the final concentration of secondary antibodies ranging from 1 to 2 µg/mL. After a 20 min incubation on the shaker, the microtiter plates were washed by vacuum filtration and 100 µL of 10 µg/mL of streptavidin-conjugated R-phycoerythrin (Molecular Probes, Eugene, OR) were added to each well. After 10 min of incubation and a final wash step, the microspheres were resuspended in 100 µL of PBST in the 96-well microtiter plate which was then placed in a Luminex 100 instrument with XY platform. The microspheres were then counted and analyzed. The amount of cytokine bound to the microsphere by this antibody-sandwich technique was determined by median fluorescence intensity of the reporter molecule. When excited at 532 nm, phycoerythrin emits at 575 nm, while the two different dyes used to label the microspheres by Luminex Corporation are excited by 635 nm laser and emit at different wave lengths of 658 and 712 nm. The ratio of the dyes can be used to identify the beads of up to 100 different fluorescent profiles. The Luminex 100 analyzer classifies each microsphere according to its predefined fluorescent emission while the third fluorophore coupled to the reporter molecular allows quantitation of the component.

### Results

Table 3 shows the mean values for the blood plasma levels of the biomarkers in the normal healthy control sample (n=109) and the MS patient sample (n=647). Table 3 also shows the p-values calculated while comparing the biomarker levels of the sample populations. As shown by Table 3, there are significant differences between the blood plasma levels of the MS and control populations for IFN-γ (p=.0019), IL-2 (p=0.0005), IL-4 (p=<.0001), IL-13 (p=<.0001), IL-1β (p=<.0001), I L-8 (p=>.0001), TNF-α (p=0.0142), CD-40L (p=<.0001), and IL-2r (p=0.0121). The results suggest that the blood plasma levels of certain cytokines and immunomodulating agents are different among normal MS patient populations.

**Table 3**

| **Biomarkers** | **Control Mean (pg/mL)** | **MS Mean (pg/mL)** | **Control Median (pg/mL)** | **MS Median (pg/mL)** | **Control Std Dev** | **MS Std Dev** | **p-value** |
|---|---|---|---|---|---|---|---|
| **(TH1 Cytokines)** | | | | | | | |
| IFN-γ | 0.197 | 5.7 | 0 | 0 | 1.2087 | 46.22 | 0.0019 |
| IL-12 | 1.429 | 8.62 | 0 | 0 | 5.457 | 62.69 | 0.0661 |
| IL-2 | 1.575 | 7.31 | 0 | 0 | 7.271 | 51.41 | 0.0005 |
| **(TH2 Cytokines)** | | | | | | | |
| IL-4 | 0.096 | 2.79 | 0 | 0.02 | 0.3249 | 40.33 | <.0001 |
| IL-5 | 5.3288 | 4.04 | 0 | 0 | 36.73 | 41.38 | 0.5559 |
| IL-10 | 10.74 | 18.44 | 1.18 | 2.52 | 45.3 | 85.21 | 0.0893 |
| IL-13 | 0.8459 | 5.72 | 0 | 0 | 3.447 | 58.38 | <.0001 |
| **(Monokines)** | | | | | | | |
| IL-1β | 13.5344 | 35.91 | 0 | 132.34 | 40.65 | 2.74 | <.0001 |
| IL-6 | 4.041 | 12.61 | 0 | 0 | 28.32 | 80.09 | 0.4841 |
| IL-8 | 4.5488 | 3.09 | 2.43 | 0 | 5.959 | 20.31 | <.0001 |
| TNF-α | 1.25 | 2.47 | 0 | 0 | 10.53 | 23.24 | 0.0142 |
| **(Immuno-modulating agents)** | | | | | | | |
| CD-40L | 83.95 | 61.1 | 54.54 | 15.83 | 89.39 | 157.09 | <.0001 |
| IL-2r | 502.455 | 463.03 | 236.373 | 416.68 | 503.47 | 410.88 | 0.0121 |

Table 4 shows the mean values for the blood plasma levels of the biomarkers in a normal healthy control sample (n=97) and a MS patient sample stratified according to clinical disease state, PP (N=16), RR (n=371), and SP (n=104).

**Table 4**

| **Biomarkers** | **Control Mean (pg/mL)** | **PP Mean (pg/mL)** | **RR Mean (pg/mL)** | **SP Mean (pg/mL)** | **Control Std Dev** | **PP Std Dev** | **RR Std Dev** | **SP Std Dev** |
|---|---|---|---|---|---|---|---|---|
| IFN-γ | 0.1 | 2.62 | 5.96 | 2.84 | 0.43 | 7.19 | 55.59 | 20.99 |
| IL-12 | 1.2 | 0.35 | 10.64 | 5.82 | 4.19 | 0.89 | 77.85 | 47.31 |
| IL-2 | 1.06 | 0.35 | 8.3 | 2.97 | 3.38 | 0.98 | 60.75 | 14.8 |
| IL-4 | 0.1 | 0.14 | 3.75 | 0.99 | 0.34 | 0.33 | 52.21 | 7.48 |
| IL-5 | 2.14 | 0.84 | 5.26 | 2.99 | 10.05 | 2.26 | 53.12 | 20.39 |
| IL-10 | 7.46 | 3.4 | 18.24 | 10.08 | 20.55 | 3.93 | 83.89 | 31.65 |
| IL-13 | 0.84 | 1.5 | 7.53 | 5.15 | 3.6 | 2.91 | 74.46 | 37.18 |
| IL-1β | 13.74 | 7.1 | 36.53 | 22.58 | 38.63 | 13.97 | 135.46 | 100.86 |
| IL-6 | 4.13 | 5.68 | 8.48 | 5.93 | 29.98 | 14.31 | 63.57 | 24.11 |
| IL-8 | 4.89 | 2.39 | 1.93 | 1.78 | 6.18 | 5.25 | 7.33 | 4.6 |
| TNF-α | 0.17 | 0.32 | 1.56 | 1.65 | 0.99 | 0.95 | 7.34 | 8.83 |
| CD-40L | 86.41 | 119.29 | 55.36 | 50.28 | 91.01 | 208.59 | 138.52 | 120.88 |
| IL-2r | 529.09 | 462.64 | 445.02 | 499.68 | 215.7 | 460.78 | 363.99 | 445.74 |

## Claims

1. A method of determining the susceptibility to multiple sclerosis (MS) in an individual, the method comprising:
providing a sample obtained from an individual in need of testing for the susceptibility of MS;
assaying the sample for the presence in the individual of at least one of: a G allele SNP at position 27 of SEQ ID NO.: 147, an A allele SNP at position 27 of SEQ ID NO.: 148, a G allele SNP at position 27 of SEQ ID NO.: 149, and an A allele SNP at position 27 of SEQ ID NO.: 150;
wherein the presence in the individual of at least one of a G allele SNP at position 27 of SEQ ID NO.: 147, an A allele SNP at position 27 of SEQ ID NO.: 148, a G allele SNP at position 27 of SEQ ID NO.: 149, and an A allele SNP at position 27 of SEQ ID NO.: 150 is indicative that the individual is susceptible to MS.

2. The method of claim 1, further comprising:
assaying for the presence or absence in the individual of at least one further biomarker associated with MS, wherein the sample is a blood plasma sample, and wherein the presence in the individual of at least one SNP as recited in claim 1 and the presence in the individual of the at least one biomarker associated with MS is indicative that the individual is susceptible to MS.

3. The method of claim 2, wherein the at least one further biomarker is selected from the group consisting of at least one anti-thyroid antibody, at least one cytokine, and at least one immunomodulating agent, or combinations thereof.

4. The method of claim 2, wherein the at least one further biomarker is at least one of TNF-α, IL-1β, IL-6, IL-8, IL-4, IL-5, IL-10, IL-13, IFN-γ, IL-2, IL-12, CD-40L and IL-2r, preferably IL-1β, IL-2, IL-6, TNF-α, and IL-4.

5. The method of claim 4, wherein assaying for the presence of the at least one further biomarker in the individual comprises assaying the blood plasma level of at least one of TNF-α, IL-1β, IL-6, IL-8, IL-4, IL-5, IL-10, IL-13, IFN-γ, IL-2, IL-12, CD-40L and IL-2r, at a level approximately greater than the mean blood plasma level of the at least one biomarker in a normal healthy control population.

6. The method of claim 2, further comprising assaying the sample for the presence of at least one of: a G allele SNP at position 27 of SEQ ID NO.: 155, and a C allele SNP at position 27 of SEQ ID NO.: 156, and wherein the at least one further biomarker is IL-4, wherein the presence in an individual of a G allele at position 27 of SEQ ID NO.: 155 and/or a C allele at position 27 of SEQ ID.: 156 is indicative that the individual is susceptible to MS.

7. The method of claim 2, wherein the at least one further biomarker is IL-1β and is detected at a blood plasma level greater than the mean blood plasma level of IL-1β in a normal healthy control population.

8. The method of claim 7 wherein IL-1β is detected at a blood plasma level of approximately 35 pg/mL.

9. The method of claim 2, wherein the at least one further biomarker is IL-2 and is detected at a blood plasma level ranging from greater than the mean blood plasma level of IL-2 in a normal healthy control population.

10. The method of claim 9 wherein IL-2 is detected at a blood plasma level of approximately 7 pg/mL.

11. The method of claim 2, wherein the at least one further biomarker is IL-6 and is detected at a blood plasma level ranging from greater than the mean blood plasma level of IL-6 in a normal healthy control population.

12. The method of claim 11 wherein IL-6 is detected at a blood plasma level of approximately 12 pg/mL.

13. The method of claim 2, wherein the at least one further biomarker is TNF-α and is detected at a blood plasma level ranging from greater than the mean blood plasma level of TNF-α in a normal healthy control population.

14. The method of claim 13 wherein TNF-α is detected at a blood plasma level of approximately 2 pg/mL.

15. The method of claim 6, wherein IL-4 is detected at a blood plasma level ranging from greater than the mean blood plasma level of TNF-α in a normal healthy control population.

16. The method of claim 15 wherein IL-4 is detected at a blood plasma level of approximately 3 pg/mL.

17. An in vitro diagnostic kit for indicating the susceptibility to multiple sclerosis (MS) in an individual using a method according to claim 1, wherein the kit comprises a product for the specific detection of the presence in the individual of at least one of: a G allele SNP at position 27 of SEQ ID NO.: 147, an A allele SNP at position 27 of SEQ ID NO.: 148, a G allele SNP at position 27 of SEQ ID NO.: 149, and an A allele SNP at position 27 of SEQ ID NO.: 150; and
a product for the specific detection of the presence in the individual of at least one further biomarker associated with MS; wherein the at least one further biomarker is at least one of TNF-α, IL-1β, IL-6, IL-8, IL-4, IL-5, IL-10, IL-13, IFN-γ, IL-2, IL-12, CD-40L, IL-2r, anti-thyroid peroxidise (anti-TPO) antibody and anti-thyroglobulin (anti-TG) antibody.

## Patentansprüche

1. Verfahren zur Bestimmung der Anfälligkeit für Multiple Sklerose (MS) bei einem Individuum, wobei das Verfahren umfasst:
Bereitstellen einer Probe, das von einem Individuum erhalten wurde, für das die Prüfung auf Anfälligkeit für (MS) notwendig ist;
Testen der Probe auf das Vorhandensein bei dem Individuum von mindestens einem von :
einem G Allel eines SNP bei Position 27 von SEQ ID NO. 147; einem A Allel eines SNP bei Position 27 von SEQ ID NO. 148; einem G Allel eines SNP bei Position 27 von SEQ ID NO. 149 und einem A Allel eines SNP bei Position 27 von SEQ ID NO. 150;
wobei das Vorhandensein in dem Individuum von mindestens einem von einem G Allel eines SNP bei Position 27 von SEQ ID NO. 147; einem A Allel eines SNP bei Position 27 von SEQ ID NO. 148; einem G Allel eines SNP bei Position 27 von SEQ ID NO. 149 und einem A Allel eines SNP bei Position 27 von SEQ ID NO. 150 anzeigt, dass das Individuum eine Anfälligkeit für MS aufweist.

2. Verfahren nach Anspruch 1, weiterhin umfassend: Testen auf die Anwesenheit oder Abwesenheit in dem Individuum von mindestens einem weiteren Biomarker, der mit MS assoziiert ist, wobei die Probe eine Blutplasmaprobe ist, wobei das Vorhandensein in dem Individuum von mindestens einem Allel eines SNP, wie ein Anspruch 1 beschrieben, und die Anwesenheit in dem Individuum eines Biomarkers, der mit MS assoziiert ist, anzeigt, dass das Individuum für MS anfällig ist.

3. Verfahren nach Anspruch 2, wobei der wenigstens eine weitere Biomarker aus der Gruppe ausgewählt ist, bestehend aus mindestens einem Anti-Schilddrüsen-Antikörper, mindestens einem Cytokin und mindestens einem immunmodulierenden Mittel oder Kombinationen davon.

4. Verfahren nach Anspruch 2, wobei der wenigstens eine weitere Biomarker mindestens einer ist von TNF-α, IL-1β, IL-6, IL-8, IL-4, IL-5, IL-10, IL-13, IFN-γ, IL-2, IL-12, CD-40L und IL-2r, vorzugsweise IL-1-β, IL-2, IL-6, TNF-α und IL-4.

5. Verfahren nach Anspruch 4, wobei das Testen auf die Anwesenheit des wenigstens einen weiteren Biomarkers in dem Individuum das Testen des Blutplasmaspiegel von mindestens einem von TNF-α, IL-1-β, IL-6, IL-8, IL-4, IL-5, IL-10, IL-13, IFN-γ, IL-2, IL-12, CD-40L und IL-2r auf einem Niveau etwa größer als der mittlere Blutplasmaspiegel des mindestens einen Biomarker in einer normalen, gesunden Kontrollpopulation umfasst.

6. Verfahren nach Anspruch 2, weiterhin umfassend das Testen der Probe auf die Anwesenheit von mindestens einem von einem G Allel eines SNP bei Position 27 von SEQ ID NO. 155 und einem C Allel eines SNP bei Position 27 von SEQ ID NO. 156, und wobei der mindestens eine weitere Biomarker IL-4 ist, wobei die Gegenwart in dem Individuum von einem G Allel eines SNP bei Position 27 von SEQ ID NO. 155 und/oder einem C Allel eines SNP bei Position 27 von SEQ ID NO. 156 anzeigt, dass das Individuum eine Anfälligkeit für MS aufweist.

7. Verfahren nach Anspruch 2, wobei der mindestens eine weitere Biomarker IL-1β ist und mit einem Blutplasmaspiegel größer als der mittlere Blutplasmaspiegel von IL-1β in einer normalen, gesunden Kontrollpopulation nachgewiesen wird.

8. Verfahren nach Anspruch 7, wobei IL-1β mit einem Blutplasmaspiegel von ungefähr 35 pg/ml nachgewiesen wird.

9. Verfahren nach Anspruch 2, wobei der mindestens eine weitere Biomarker IL-2 ist und mit einem Blutplasmaspiegel von größer als der mittlere Blutplasmaspiegel von IL-2 in einer normalen, gesunden Kontrollpopulation nachgewiesen wird.

10. Verfahren nach Anspruch 9, wobei IL-2 mit einem Blutplasmaspiegel von ungefähr 7 pg/ml nachgewiesen wird.

11. Verfahren nach Anspruch 2, wobei der mindestens eine weitere Biomarker IL-6 ist und mit einem Blutplasmaspiegel von größer als der mittlere Blutplasmaspiegel von IL-6 in einer normalen, gesunden Kontrollpopulation nachgewiesen wird.

12. Verfahren nach Anspruch 11, wobei IL-6 mit einem Blutplasmaspiegel von ungefähr 12 pg/ml nachgewiesen wird.

13. Verfahren nach Anspruch 2, wobei der mindestens eine weitere Biomarker TNF-α ist und mit einem Blutplasmaspiegel von größer als der mittlere Blutplasmaspiegel von TNF-α in einer normalen, gesunden Kontrollpopulation nachgewiesen wird.

14. Verfahren nach Anspruch 13, wobei TNF-α mit einem Blutplasmaspiegel von ungefähr 2 pg/ml nachgewiesen wird.

15. Verfahren nach Anspruch 6, wobei IL-4 mit einem Blutplasmaspiegel von größer als der mittlere Blutplasmaspiegel von TNF-α in einer normalen, gesunden Kontrollpopulation nachgewiesen wird.

16. Verfahren nach Anspruch 15, wobei IL-4 mit einem Blutplasmaspiegel von ungefähr 3 pg/ml nachgewiesen wird.

17. Ein in vitro Diagnosekit zur Bestimmung der Anfälligkeit für Multiple Sklerose (MS) bei einem Individuum unter Verwendung eines Verfahrens gemäß Anspruch 1, wobei das Kit ein Produkt zur spezifischen Bestimmung des Vorhandenseins in dem Individuum von mindestens einem von: einem G Allel eines SNP bei Position 27 von SEQ ID NO. 147; einem A Allel eines SNP bei Position 27 von SEQ ID NO. 148; einem G Allel eines SNP bei Position 27 von SEQ ID NO. 149 und einem A Allel eines SNP bei Position 27 von SEQ ID NO. 150; und ein Produkt zur spezifischen Bestimmung des Vorhandenseins in dem Individuum von mindestens einem weiteren Biomarker, der mit MS assoziiert ist, wobei der wenigstens eine weitere Biomarker mindestens einer ist von TNF-α, IL-1β, IL-6, IL-8, IL-4, IL-5, IL-10, IL-13, IFN-γ, IL-2, IL-12, CD-40L und IL-2r, Anti-Thyreoperoxidase (anti-TPO) Antikörper und einem anti-Thyreoglobulin (anti-TG) Antikörper, umfasst.

## Revendications

1. Procédé de détermination de la prédisposition à la sclérose en plaques (SEP) chez un individu, le procédé comprenant les étapes consistant à :
fournir un échantillon obtenu d'un individu ayant besoin d'être testé quant à la prédisposition à la SEP ;
doser l'échantillon pour détecter la présence chez l'individu d'au moins un parmi : un SNP à allèle G dans la position 27 de SEQ ID NO.: 147, un SNP à allèle A dans la position 27 de SEQ ID NO.: 148, un SNP à allèle G dans la position 27 de SEQ ID NO.: 149, et un SNP à allèle A dans la position 27 de SEQ ID NO.: 150 ;
dans lequel la présence chez l'individu d'au moins un parmi un SNP à allèle G dans la position 27 de SEQ ID NO.: 147, un SNP à allèle A dans la position 27 de SEQ ID NO.: 148, un SNP à allèle G dans la position 27 de SEQ ID NO.: 149, et un SNP à allèle A dans la position 27 de SEQ ID NO.: 150 indique que le individu est prédisposé à la sclérose en plaques.

2. Procédé selon la revendication 1, comprenant en outre l'étape consistant à :
doser pour la présence ou l'absence chez l'individu d'au moins un biomarqueur supplémentaire associé à la SEP, dans lequel l'échantillon est un échantillon de plasma sanguin, et dans lequel la présence chez l'individu d'au moins un SNP, tel que défini dans la revendication 1, et la présence chez l'individu d'au moins un marqueur biologique associé à la SEP est une indication que le individu est prédisposé à la SEP.

3. Procédé selon la revendication 2, dans lequel le au moins un biomarqueur supplémentaire est choisi dans le groupe constitué par au moins un anticorps anti-thyroïdien, au moins une cytokine, et au moins un agent immunomodulateur, ou des combinaisons de ceux-ci.

4. Procédé selon la revendication 2, dans lequel le au moins un biomarqueur supplémentaire est au moins un parmi TNF-α, IL-1β, IL-6, IL-8, IL-4, IL-5, IL-10, 1L-13, IFN-γ, IL-2, IL-12, CD-40L et IL-2r, de préférence IL-1β, IL-2, IL-6, TNF-α et IL-4.

5. Procédé selon la revendication 4, dans lequel un dosage pour la présence du au moins un biomarqueur supplémentaire chez l'individu comprend un dosage du niveau de plasma sanguin d'au moins un parmi TNF-α, IL-1β, IL-6, IL-8, IL-4, IL-5, IL-10, 1L-13, IFN-γ, IL-2, IL-12, CD-40L et IL-2r, à un niveau sensiblement supérieur au niveau de plasma sanguin moyen du au moins un biomarqueur dans une population témoin normale et saine.

6. Procédé selon la revendication 2, comprenant en outre le dosage de l'échantillon pour détecter la présence d'au moins un parmi : un SNP à allèle G dans la position 27 de SEQ ID NO.: 155, et un SNP à allèle C dans la position 27 de SEQ ID NO.: 156, dans lequel la présence chez un individu d'un SNP à allèle G dans la position 27 de SEQ ID NO.: 155 et/ou d'un SNP à allèle C dans la position 27 de SEQ ID NO.: 156 est une indication que le individuel est prédisposé à la SEP.

7. Procédé selon la revendication 2, dans lequel le au moins un biomarqueur supplémentaire est IL-1β, et est détecté à un niveau de plasma sanguin supérieur au niveau de plasma sanguin moyen de IL-1βdans une population témoin saine et normale.

8. Procédé selon la revendication 7, dans lequel IL-1β est détecté à un niveau de plasma sanguin d'environ 35 pg/ml.

9. Procédé selon la revendication 2, dans lequel le au moins un biomarqueur supplémentaire est IL-2, et est détecté à un niveau de plasma sanguin partant de plus que le niveau de plasma sanguin moyen d'IL-2 dans une population témoin saine et normale.

10. Procédé selon la revendication 9, dans lequel IL-2 est détecté à un niveau de plasma sanguin d'environ 7 pg/ml.

11. Procédé selon la revendication 2, dans lequel le au moins un biomarqueur supplémentaire est IL-6, et est détecté à un niveau de plasma sanguin partant de plus que le niveau de plasma sanguin moyen d'IL-6 dans une population témoin saine et normale.

12. Procédé selon la revendication 11, dans lequel IL-6 est détecté à un niveau de plasma sanguin d'environ 12 pg/ml.

13. Procédé selon la revendication 2, dans lequel le au moins un biomarqueur supplémentaire est TNF-α, et est détecté à un niveau de plasma sanguin partant de plus que le niveau de plasma sanguin moyen de TNF-α dans une population témoin saine et normale.

14. Procédé selon la revendication 13, dans lequel TNF-α est détecté à un niveau de plasma sanguin d'environ 2 pg/ml.

15. Procédé selon la revendication 6, dans lequel IL-4 est détecté à un niveau de plasma sanguin partant de plus que le niveau de plasma sanguin moyen de TNF-α dans une population témoin saine et normale.

16. Procédé selon la revendication 15, dans lequel IL-4 est détecté à un niveau de plasma sanguin d'environ 3 pg/ml.

17. Kit dans de diagnostic in vitro pour indiquer la prédisposition à la sclérose en plaques (SEP) d'un individu en utilisant un procédé selon la revendication 1, dans lequel le kit comprend un produit pour la détection spécifique de la présence chez l'individu d'au moins un parmi : un SNP à allèle G dans la position 27 de SEQ ID NO.: 147, un SNP à allèle A dans la position 27 de SEQ ID NO.: 148, un SNP à allèle G dans la position 27 de SEQ ID NO.: 149, et un SNP à allèle A dans la position 27 de SEQ ID NO.: 150; et
un produit pour la détection spécifique de la présence chez l'individu d'au moins un autre biomarqueur associé à la SEP ; dans lequel le au moins un autre biomarqueur est au moins un parmi TNF-α, IL-1β, IL-6, IL-8, IL-4, IL-5, IL-10, 1L-13, IFN-γ, IL-2, IL-12, CD-40L et IL-2r, un anticorps peroxydase thyroïdienne (anti-TPO) et un anticorps anti-thyroglobuline (anti-TG).
